(19) Europäisches Patentamt
European Patent Office
Office européen des brevets

(11) Veröffentlichungsnummer : **0 049 711**
**B1**

(12) **EUROPÄISCHE PATENTSCHRIFT**

(45) Veröffentlichungstag der Patentschrift : **29.02.84**

(51) Int. Cl.³ : **C 07 C 93/14**, C 07 C 87/60, C 07 C 85/24

(21) Anmeldenummer : **80106383.5**

(22) Anmeldetag : **20.10.80**

(54) **Verfahren zur Herstellung von Nitroaminobenzolen.**

(30) Priorität : **10.10.80 DE 3038394**

(43) Veröffentlichungstag der Anmeldung : **21.04.82 Patentblatt 82/16**

(45) Bekanntmachung des Hinweises auf die Patenterteilung : **29.02.84 Patentblatt 84/09**

(84) Benannte Vertragsstaaten : **BE CH DE FR GB IT LI SE**

(56) Entgegenhaltungen :
**DE-A- 2 226 405**
**US-A- 1 963 598**
**US-A- 2 525 508**

(73) Patentinhaber : **BAYER AG**
**Zentralbereich Patente, Marken und Lizenzen**
**D-5090 Leverkusen 1, Bayerwerk (DE)**

(72) Erfinder : **Schössler, Willi, Dr.**
**Hahnenweg 2**
**D-5000 Koeln 80 (DE)**
Erfinder : **Blank, Heinz-Ulrich, Dr.**
**Am Geusfelde 35**
**D-5068 Odenthal (DE)**
Erfinder : **Dürholz, Friedrich, Dr.**
**Knusthöhe 40**
**D-5630 Remscheid 11 (DE)**

**EP 0 049 711 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von Nitroaminobenzolen

Die vorliegende Erfindung betrifft ein Verfahren zur Herstellung von Nitroaminobenzolen aus am Stickstoff geschützten Aminobenzolen durch Nitrierung in Gegenwart von inerten, mit Wasser nicht mischbaren organischen Lösungs- und/oder Verdünnungsmitteln.

Es ist bekannt, Nitroacylaminobenzole und die entsprechenden Nitroaminobenzole dadurch herzustellen, daß man in einem Eintopfverfahren ausgehend von Aminobenzolen diese zuerst mit Acylierungsmitteln, wie Acetanhydrid, Benzoylchlorid oder p-Toluolsulfonsäurechlorid, in Gegenwart von inerten organischen Lösungsmitteln, bevorzugt in Monochlorbenzol, bei erhöhter Temperatur umsetzt und anschließend ohne Zwischenisolierung bei 25 bis 50 °C nitriert (vgl. US-PS 2 459 002, Beispiele 1 bis 8).

Nachteilig bei dem in der US-PS beschriebenen Verfahren sind die geringen Ausbeuten und die schlechten Qualitäten an Nitroaminobenzolen, wie Nacharbeitungen der Beispiele 2 und 4 der US-PS zeigen (in den Beispielen der US-PS fehlt nämlich eine Angabe zur Ausbeute und Produktqualität).

Da bei dem Verfahren der US-PS 2 459 002 die Acylierung und Nitrierung nacheinander, d. h. ohne Isolierung des acylierten Zwischenprodukts durchgeführt werden, besteht das Nitriergemisch immer aus überschüssigem Acylierungsmittel, freigesetzter organischer oder anorganischer Säure und Salpetersäure. Dies hat jedoch den Nachteil, daß neben den geringen Ausbeuten und der schlechten Qualitäten an Nitroaminobenzolen bei der anschließenden Neutralisation des Reaktionsgemisches mehr Alkali benötigt wird als eigentlich für die Neutralisation der Salpetersäure erforderlich ist. Durch die Neutralisation geht das überschüssige Acylierungsmittel und die freigesetzte organische oder anorganische Säure für eine weitere wirtschaftliche Nutzung verloren und zusätzlich wird das Abwasser durch die bei der Neutralisation gebildeten Salze belastet.

Ein weiterer Nachteil des in der US-PS geschilderten Verfahrens besteht darin, daß, wenn ohne Zwischenisolierung der Nitroacylaminoverbindungen die anschließende Schutzgruppenabspaltung in dem zuvor neutralisierten und vom Chlorbenzol befreiten Nitriergemisch in saurem Medium durchgeführt wird, große Mengen an Schwefelsäure erforderlich sind (vgl. hierzu US-PS 2 459 002, Beispiele 4 und 7, wo pro Mol Aminobenzol 6,6 bzw. 4,4 Mol Schwefelsäure eingesetzt werden). Da die Schutzgruppenabspaltung bei diesem Verfahren zudem in konzentrierter Schwefelsäure durchgeführt wird, ist es erforderlich, daß die Schwefelsäure zur Isolierung der Nitroaminobenzole zunächst mit Wasser verdünnt und anschließend mit Soda neutralisiert wird. Hierbei fallen große Mengen an salzhaltigen Abwässern an, die mit beträchtlichem Aufwand beseitigt bzw. aufgearbeitet werden müssen.

Weiterhin wurde bei der Nacharbeitung der Beispiele 2 und 4 der US-PS 2 459 002 beobachtet, daß, wenn das 5-Nitro-2-acetylaminoanisol bzw. das 3-Nitro-4-acetylaminotoluol ohne Zwischenisolierung in saurem Medium gespalten wird, zum Teil Verharzung eintritt. Zur Isolierung des 5-Nitro-2-aminoanisols bzw. des 3-Nitro-4-aminotoluols ist es daher erforderlich, die Reaktionslösung zuvor mit Aktivkohle zu behandeln.

Es ist ferner bekannt, 3-Nitro-4-aminoanisol herzustellen, indem man ausgehend von 4-Acetylaminoanisol dieses simultan mit ca. 61,5 %iger, wäßriger Salpetersäure innerhalb von ca. 3 Stunden in vorgelegtes Monochlorbenzol, welches bereits einen Teil der zur Nitrierung erforderlichen Salpetersäure sowie größere Mengen an Kochsalz enthält, bei 20 bis 30 °C eindosiert und das ausreagierte Nitriergemisch ohne Zwischenisolierung des 3-Nitro-4-acetylaminoanisols nach Neutralisation der überschüssigen Salpetersäure und Wasserdampfdestillation des Monochlorbenzols alkalisch spaltet (vgl. Bios Final Report Nr. 986, Seite 285-288).

Ein wesentlicher Nachteil dieses Verfahrens ist darin zu sehen, daß die Nitrierung in Gegenwart großer Mengen Kochsalz durchgeführt wird, wodurch das anfallende Abwasser zusätzlich belastet wird. Eine weitere Belastung des Abwassers entsteht durch das bei der Neutralisation der überschüssigen Salpetersäure gebildete Natriumnitrat.

Nachteilig ist weiterhin, daß das Chlorbenzol aus dem zuvor mit Soda schwach alkalisch gestellten Nitriergemisch vor der alkalischen Abspaltung der Schutzgruppe durch eine ca. 5 Stunden dauernde Wasserdampfdestillation entfernt wird, was Energie aufwendig ist. Unbefriedigend sind ferner die erzielten Ausbeuten und Qualitäten an 3-Nitro-4-aminoanisol.

Weiterhin ist aus US 2.525.508 ein Verfahren zur Nitrierung in meta-Stellung von ortho-substituierten Aminen mit freier Aminogruppe bekannt, bei dem in Schwefelsäure als Reaktionsmedium gearbeitet wird und das Substrat und die $HNO_3/H_2SO_4$-Nitriersäure simultan in vorgelegte $H_2SO_4$ eindosiert werden. Diese Verfahrensweise soll geeignet sein, die unerwünschte Sulfonierung des Substrats zu verhindern. Um die ebenfalls unerwünschte Zweitnitrierung zu unterdrücken, wird mit äquivalenten Mengen $HNO_3$, bezogen auf das Substrat gearbeitet.

Es wurde nun ein Verfahren zur Herstellung von Nitroaminobenzolen durch Nitrieren von am Stickstoff geschützten Aminobenzolen mit Salpetersäure in Gegenwart von inerten, mit Wasser nicht mischbaren organischen Lösungs- und/oder Verdünnungsmitteln, gegebenenfalls in Gegenwart von salpetriger Säure und/oder Salpetrigsäure bildenden Substanzen sowie gegebenenfalls in Gegenwart von wasserbindenden Mitteln, bei Temperaturen von 0 bis 80 °C gefunden, das dadurch gekennzeichnet ist, daß man unter gleichzeitiger Zugabe von 50 bis 100 gew.-%iger Salpetersäure und der zu nitrierenden

Verbindung in Gegenwart von Methylenchlorid nitriert, anschließend die Schutzgruppe der Nitroaminobenzole, gegebenenfalls nach vorheriger Abtrennung des Methylenchlorids, abspaltet und die Nitroaminobenzole isoliert.

In einer bevorzugten Ausführungsform des erfindungsgemäßen Verfahrens wird nach beendeter Nitrierung die überschüssige Salpetersäure ganz oder teilweise aus dem Nitriergemisch, gegebenenfalls nach vorheriger Neutralisation der Salpetersäure, entfernt, anschließend die Schutzgruppe der Nitroaminobenzole, gegebenenfalls nach vorheriger Abtrennung des Methylenchlorids, abgespalten und die Nitroaminobenzole isoliert.

In einer besonders bevorzugten Verfahrenweise des erfindungsgemäßen Verfahrens wird nach beendeter Nitrierung die überschüssige Salpetersäure ganz oder teilweise aus dem Nitriergemisch, gegebenenfalls nach vorheriger Neutralisation der Salpetersäure und gegebenenfalls nach einer Phasentrennung, durch Extraktion mit Wasser entfernt.

Als am Stickstoff geschützte Aminobenzole können solche der allgemeinen Formel (I)

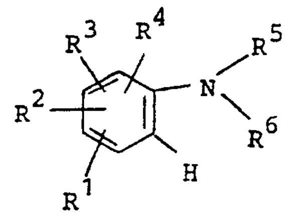

(I)

worin

$R^1$ bis $R^4$ gleich oder verschieden sind und für 1 bis 4 Wasserstoffatome, 1 bis 4 Halogenatome, 1 bis 4 Alkylreste, 1 bis 2 Alkoxyreste, 1 bis 2 Acyloxyreste, 1 bis 2 Aryloxyreste, einen Acylamino-, einen Carboxyl-, einen Alkoxycarbonyl-, einen Alkylsulfonyl-, einen Aralkoxy-, einen Aralkylsulfonyl-, einen Arylsulfonyl-, einen Hydroxysulfonyl- oder einen Aminosulfonylrest stehen

$R^5$ Wasserstoff und

$R^6$ Formyl, Oxalyl, Alkylcarbonyl, Alkylsulfonyl, Alkoxycarbonyl, Arylcarbonyl, Arylsulfonyl oder

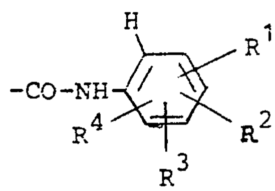

wobei

$R^1$ bis $R^4$ gleich oder verschieden sind und die o. g. Bedeutung haben, oder

$$—CO—NH—R'$$

wobei

R' Wasserstoff oder Alkyl, bevorzugt Wasserstoff, ist, bedeuten, bevorzugt $R^6$ für Alkylcarbonyl, Alkylsulfonyl, Alkoxycarbonyl, Arylcarbonyl oder Arylsulfonyl, besonders bevorzugt für Alkylcarbonyl oder Alkoxycarbonyl, steht, und wobei

$R^5$ und $R^6$ zusammen für einen Phthalylrest stehen und wobei

$R^5$ und $R^6$ gemeinsam mit einem zum durch die geschützte Aminogruppe substituierten Kohlenstoffatom ortho-ständigen Ringkohlenstoffatom einen 2-methylsubstituierten Oxazol-, einen Oxazolon- oder einen 2,6-Dioxo-dihydro-1,3-oxazinring bilden, wobei gleichzeitig in para-Stellung zu dem Ringkohlenstoffatom, das durch die geschützte Aminogruppe substituiert ist, einer der Reste $R^1$ bis $R^4$ außer Wasserstoff vorhanden sein muß, in das erfindungsgemäße Verfahren eingesetzt werden.

Bevorzugt werden in das erfindungsgemäße Verfahren Aminobenzole der allgemeinen Formel (II)

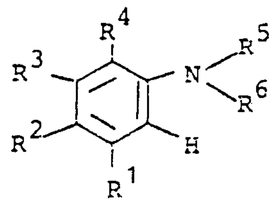

(II)

worin

$R^1$ für Wasserstoff, Halogen, Alkyl oder Alkoxy bevorzugt für Halogen, Alkyl oder Alkoxy, steht ;

$R^2$ für Wasserstoff, Halogen, Alkyl, Alkoxy, Acyloxy, Acylamino, Alkyl- und Arylsulfonyl, Aryloxy, Carboxyl oder Hydroxysulfonyl, bevorzugt für Halogen, Alkyl, Alkoxy, Acyloxy, Acylamino, Alkylsulfonyl oder Arylsulfonyl, steht ;

$R^3$ für Wasserstoff, Halogen, Alkyl, Alkoxy, Acyloxy oder Acylamino, bevorzugt für Halogen, Alkyl, Alkoxy, Acyloxy oder Acylamino, steht ;

$R^4$ für Wasserstoff, Halogen, Alkyl, Alkoxy, Aryloxy oder Carboxyl, bevorzugt für Wasserstoff, Halogen, Alkyl oder Alkoxy, steht ;

$R^5$ Wasserstoff bedeutet und

$R^6$ für Formyl, Oxalyl, Alkylcarbonyl, Alkylsulfonyl, Alkoxycarbonyl, Arylcarbonyl, Arylsulfonyl oder

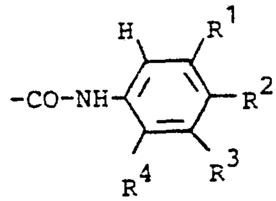

wobei

$R^1$ bis $R^4$ gleich oder verschieden sind und die zuvor genannte Bedeutung haben, oder

—CO—NH—R'

wobei

R' Wasserstoff oder Alkyl, bevorzugt Wasserstoff, ist, steht, bevorzugt

$R^6$ für Alkylcarbonyl, Alkylsulfonyl, Alkoxycarbonyl, Arylcarbonyl oder Arylsulfonyl, besonders bevorzugt für Alkylcarbonyl oder Alkoxycarbonyl, steht und wobei

$R^5$ und $R^6$ zusammen für einen Phthalylrest stehen und wobei

$R^5$ und $R^6$ gemeinsam mit einem zum durch die geschützte Aminogruppe substituierten Kohlenstoffatom ortho-ständigen Ring Kohlenstoffatom einen 2-methylsubstituierten Oxazol-, einen Oxazolon- oder einen 2,6-Dioxo-dihydro-1,3-oxazinring bilden, wobei gleichzeitig in para-Stellung zu dem durch die geschützte Aminogruppe substituierten Ringkohlenstoffatom einer der Reste $R^1$ bis $R^4$ außer Wasserstoff vorhanden sein muß, eingesetzt.

Als am Stickstoff geschützte Aminobenzole können weiterhin solche der allgemeinen Formel (III)

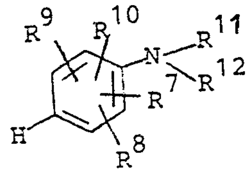

(III)

worin

$R^7$ bis $R^{10}$ gleich oder verschieden sind und für 1 bis 4 Wasserstoffatome, 1 bis 4 Halogenatome, 1 bis 4 Alkylreste, 1 bis 2 Alkoxyreste, 1 bis 2 Acyloxyreste, 1 bis 2 Aryloxyreste, einen Acylamino-, einen Carboxyl-, einen Alkoxycarbonyl-, einen Alkylsulfonyl-, einen Aralkoxy-, einen Aralkylsulfonyl-, einen Arylsulfonyl-, einen Hydroxysulfonyl- oder einen Aminosulfonylrest stehen,

$R^{11}$ Wasserstoff und

$R^{12}$ Formyl, Oxalyl, Alkylcarbonyl, Alkylsulfonyl, Alkoxycarbonyl, Arylcarbonyl, Arylsulfonyl oder

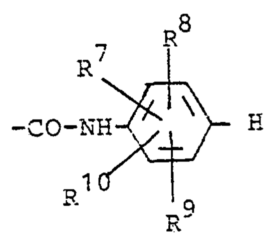

wobei

$R^7$ bis $R^{10}$ gleich oder verschieden sind und die zuvor genannte Bedeutung haben, oder

—CO—NH—R'

wobei

R' für Wasserstoff oder Alkyl, bevorzugt für Wasserstoff, steht, bedeuten, bevorzugt

$R^{12}$ für Alkylcarbonyl, Alkylsulfonyl, Alkoxycarbonyl, Arylcarbonyl oder Arylsulfonyl, besonders bevorzugt für Alkylcarbonyl oder Alkoxycarbonyl, steht und wobei

$R^{11}$ und $R^{12}$ zusammen für einen Phthalylrest stehen, und wobei

$R^{11}$ und $R^{12}$ gemeinsam mit einem zum durch die geschützte Aminogruppe substituierten Kohlenstoffatom ortho-ständigen Ringkohlenstoffatom einen 2-methylsubstituierten Oxazol-, einen Oxazolon- oder einen 2,6-Dioxo-dihydro-1,3-oxazinring bilden, eingesetzt werden.

Als bevorzugte Aminobenzole werden solche der allgemeinen Formel (IV)

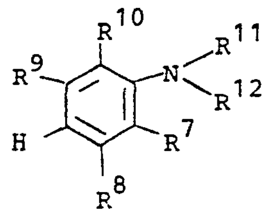

(IV)

worin

$R^7$ für Wasserstoff, Halogen, Alkyl, Alkoxy, Acyloxy, Acylamino, Carboxyl, Alkyl, Arylsulfonyl oder Aryloxy, bevorzugt für Halogen, Alkyl, Alkoxy, Acyloxy, Acylamino, Alkylsulfonyl oder Arylsulfonyl, steht ;

$R^8$ für Wasserstoff, Halogen, Alkyl, Alkoxy, Alkoxycarbonyl, Acyloxy, Acylamino, Carboxyl, Alkylsulfonyl oder Arylsulfonyl, bevorzugt für Wasserstoff, Halogen, Alkyl, Alkoxy, Acyloxy oder Acylamino, steht ;

$R^9$ für Wasserstoff, Halogen, Alkyl, Alkoxy, Alkoxycarbonyl, Acyloxy, Acylamino, Alkylsulfonyl, Arylsulfonyl oder Aralkylsulfonyl bevorzugt für Wasserstoff, Halogen, Alkyl, Alkoxy, Alkoxycarbonyl, Acyloxy oder Acylamino, steht ;

$R^{10}$ für Wasserstoff, Halogen oder Alkyl steht ;

$R^{11}$ Wasserstoff und

$R^{12}$ Formyl, Oxalyl, Alkylcarbonyl, Alkylsulfonyl, Alkoxycarbonyl, Arylcarbonyl, Acylsulfonyl oder

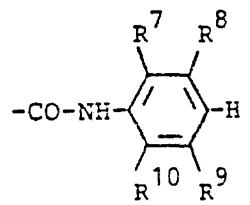

wobei

$R^7$ bis $R^{10}$ gleich oder verschieden sind und die zuvor genannte Bedeutung haben oder

—CO—NH—R'

wobei

R' für Wasserstoff oder Alkyl, bevorzugt für Wasserstoff, steht, bedeuten, bevorzugt

$R^{12}$ für Alkylcarbonyl, Alkylsulfonyl, Alkoxycarbonyl, Arylcarbonyl oder Arylsulfonyl, besonders bevorzugt für Alkylcarbonyl oder Alkoxycarbonyl, steht, und wobei

$R^{11}$ und $R^{12}$ zusammen für einen Phthalylrest stehen und wobei

$R^{11}$ und $R^{12}$ gemeinsam mit einem zum durch die geschützte Aminogruppe substituierten Kohlenstoffatom ortho-ständigen Ringkohlenstoffatom einen 2-methylsubstituierten Oxazol-, einen Oxazolon- oder einen 2,6-Dioxo-dihydro-1,3-oxazinring bilden, in das erfindungsgemäße Verfahren eingesetzt.

Als Halogene der Formeln (I) bis (IV) kommen in Frage : Fluor, Chlor oder Brom, bevorzugt Fluor oder Chlor, insbesondere Chlor ;

als Akylreste solche mit 1 bis 12, bevorzugt 1 bis 6, Kohlenstoffatomen, die gegebenenfalls durch Halogen, wie Fluor, Chlor oder Brom und/oder durch Alkoxygruppen, wie Methoxy, Ethoxy, Propoxy oder Butoxy, substituiert sein können. Zum Beispiel werden genannt : der Methyl-, Trifluormethyl-, Ethyl-, n-Propyl-, iso-Propyl-, n-Butyl-, iso-Butyl-, tert.-Butyl-, n-Pentyl-, iso-Pentyl-, n-Hexyl- und der Cyclohexylrest, bevorzugt der Methyl-, Trifluormethyl-, Ethyl-, n-Propyl-, iso-Propyl-, n-Butyl-, iso-Butyl- und der tert.-Butylrest, insbesondere der Methyl-, Trifluormethyl- und der Ethylrest.

Als Alkoxyreste der Formeln (I) bis (IV), die gegebenenfalls durch eine Carboxylgruppe substituiert sein können, seien solche mit 1 bis 6, bevorzugt 1 bis 4, Kohlenstoffatomen oder solche der Formel

$$-(OCH_2-\underset{R^{14}}{CH})_m-OR^{13}$$

wobei

$R^{13}$ einen Alkylrest mit 1 bis 6, bevorzugt 1 bis 4, Kohlenstoffatomen oder einen Acylrest mit 1 bis 7, bevorzugt 1 bis 4, Kohlenstoffatomen bedeutet und

$R^{14}$ für Wasserstoff oder Methyl, bevorzugt für Wasserstoff, steht und

m eine ganze Zahl von 1 bis 6, bevorzugt 1 bis 2 sein kann, genannt.

Zum Beispiel werden folgende Alkoxyreste genannt :

$—OCH_3$, $—OC_2H_5$, $—OC_3H_7$, $—OC_4H_9$, $—OCH_2CH_2OCH_3$, $—OCH_2CH_2OC_2H_5$, $—OCH_2CH_2OC_3H_7$, $—OCH_2CH_2OC_4H_9$, $—(OCH_2CH_2)_2OCH_3$, $—(OCH_2—CH_2)_2OC_2H_5$, $—(OCH_2CH_2)_2OC_3H_7$, $—(OCH_2CH_2)_2OC_4H_9$, $—OCH_2CH_2OCOCH_3$, $—OCH_2CH_2OCOC_2H_5$, $—OCH_2CH_2OCOC_3H_7$, $—OCH_2CH_2OCOC_4H_9$, $—(OCH_2CH_2)_2OCOCH_3$, $—(OCH_2CH_2)_2OCOC_2H_5$, $—(OCH_2CH_2)_2OCOC_3H_7$, $—(OCH_2CH_2)_2OCOC_4H_9$, $—OCH_2CH_2OCO—$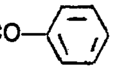, bevorzugt $—OCH_3$, $—OC_2H_5$, $—OCH_2CH_2OCH_3$, $—OCH_2CH_2OC_2H_5$, $—O(CH_2CH_2)_2OCH_3$, $—(OCH_2CH_2)_2OC_2H_5$, $—OCH_2CH_2OCOCH_3$, $—(OCH_2CH_2)_2OCOCH_3$.

Als Acyloxyreste der Formeln (I) bis (IV) seien solche genannt, bei denen der Acylrest einen Alkylcarbonylrest mit 2 bis 7, bevorzugt mit 2 bis 5, Kohlenstoffatomen, oder einen Benzoylrest, der gegebenenfalls durch Halogen, wie Fluor, Chlor oder Brom, durch eine Nitrogruppe, durch einen Methyl- oder durch einen Methoxyrest substituiert sein kann, bedeutet. Z. B. werden folgende Acyloxyreste genannt :

$-OCOCH_3$, $-OCOC_2H_5$, $-OCOC_3H_7$, $-OCOC_4H_9$, -OCO-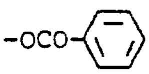,

-OCO-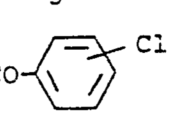, -OCO-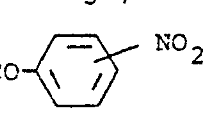,

bevorzugt

$-OCOCH_3$, $-OCOC_2H_5$, -OCO-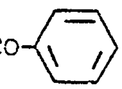, insbesondere $-OCOCH_3$.

Als Acylaminoreste der Formeln (I) bis (IV) seien solche genannt, bei denen der Acylrest einen Formylrest, einen Alkylcarbonylrest mit 2 bis 7, bevorzugt 2 bis 5, Kohlenstoffatomen oder einen Benzoylylrest, der gegebenenfalls durch Halogen, wie Fluor, Chlor oder Brom, durch eine Nitrogruppe, durch einen Methyl- oder durch einen Methoxyrest substituiert sein kann, bedeutet.

Es seien folgende Acylaminoreste genannt :

$—NHCOH$, $—NHCOCH_3$, $—NHCOC_2H_5$, $—NHCOC_3H_7$, $—NHCOC_4H_9$,

-NHCO-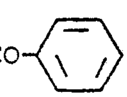, -NHCO-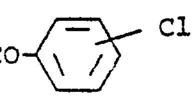, -NHCO-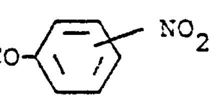,

bevorzugt

$-NHCOCH_3$, $-NHCOC_2H_5$, -NHCO-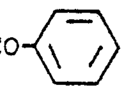, insbesondere $NHCOCH_3$;

Als Alkoxycarbonylreste der Formeln (I) bis (IV) kommen solche mit 2 bis 7, bevorzugt 2 bis 5, Kohlenstoffatomen in Frage, z. B. Methoxycarbonyl, Ethoxycarbonyl, Propoxycarbonyl, Butoxycarbonyl, bevorzugt Methoxycarbonyl und Ethoxycarbonyl ;

Als Alkylsulfonylreste der Formeln (I) bis (IV) solche mit 1 bis 6, bevorzugt 1 bis 4, Kohlenstoffatomen, wie Methylsulfonyl, Ethylsulfonyl, Propylsulfonyl, Butylsulfonyl, bevorzugt Methylsulfonyl.

Als Aralkoxyreste oder Aralkylsulfonylreste der Formeln (I) bis (IV), die gegebenenfalls ein- oder mehrfach durch Halogen, wie Fluor, Chlor oder Brom, und/oder durch eine Nitrogruppe und/oder durch eine Alkyl- und/oder durch eine Alkoxygruppe mit 1 bis 6, bevorzugt 1 bis 4, Kohlenstoffatomen substituiert sein können, kommen solche mit 7 bis 17, bevorzugt 7 bis 13, Kohlenstoffatomen in Betracht. Zum Beispiel werden genannt :

$-OCH_2$-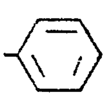, $-OC_2H_4$-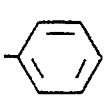, $-OC_3H_6$-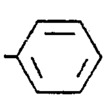, $-OC_4H_8$-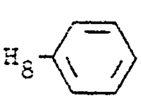,

$-OCH_2$-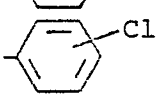, $-OCH_2$-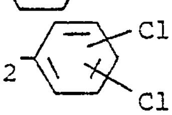, $-OCH_2$-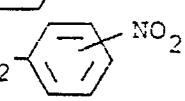,

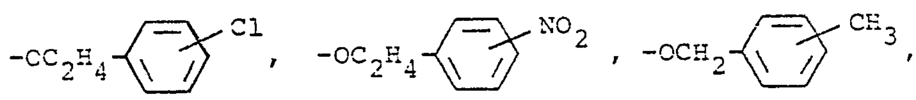

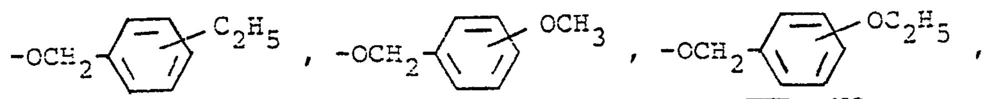

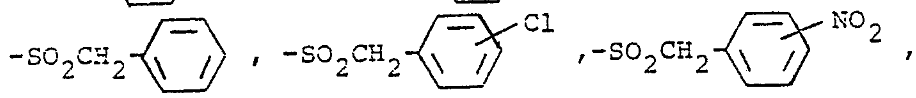

bevorzugt

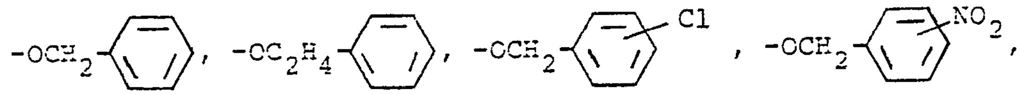

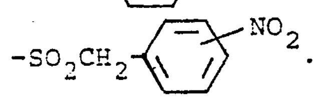

Als Aryloxy- oder Arylsulfonylreste der Formeln (I) bis (IV), die gegebenenfalls ein- oder mehrfach durch Halogen, wie Fluor, Chlor oder Brom, und/oder durch eine Nitrogruppe und/oder einen Alkyl- und/oder Alkoxyrest mit 1 bis 6, bevorzugt 1 bis 4, Kohlenstoffatomen substituiert sein können, kommen solche mit 6 bis 12, bevorzugt 6 bis 10, Kohlenstoffatomen in Frage. Es werden beispielsweise genannt :

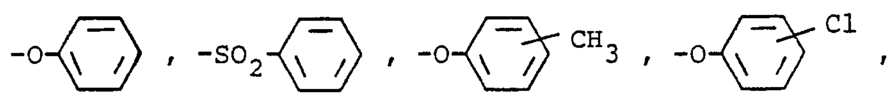

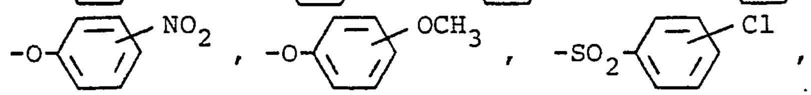

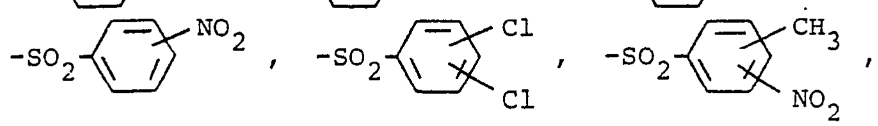

bevorzugt

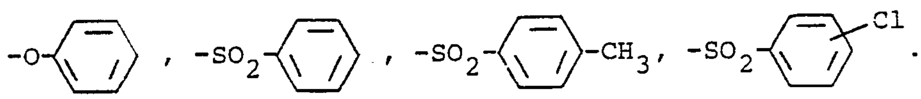

Als Alkylcarbonylreste, die gegebenenfalls ein- oder mehrfach durch Halogen, wie Fluor, Chlor oder Brom, substituiert sein können, seien solche mit 2 bis 7, bevorzugt 2 bis 5, Kohlenstoffatomen genannt, wie Acetyl, Ethylcarbonyl, Propylcarbonyl, Butylcarbonyl, Trifluoracetyl, bevorzugt Acetyl, Ethylcarbonyl, insbesondere Acetyl.

Als Arylcarbonylreste, die gegebenenfalls ein- oder mehrfach durch Halogen, wie Fluor, Chlor oder Brom, und/oder durch eine Nitrogruppe und/oder durch einen Alkyl- und/oder Alkoxyrest mit 1 bis 6, bevorzugt 1 bis 4, Kohlenstoffatomen substituiert sein können, seien solche mit 7 bis 13, bevorzugt 7 bis 11, Kohlenstoffatomen genannt, wie

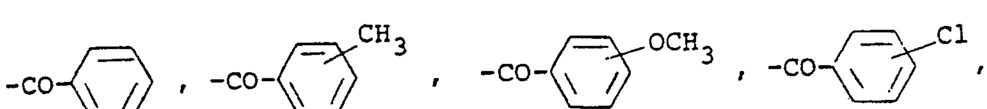

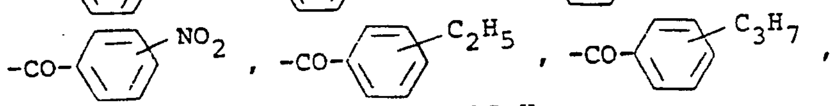

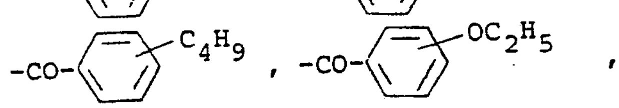

bevorzugt 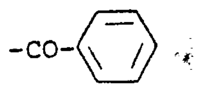

Als Aminosulfonylreste der Formeln (I) bis (IV), die gegebenenfalls durch Alkylreste mit 1 bis 6, bevorzugt 1 bis 4, Kohlenstoffatomen substituiert sein können, kommen in Frage : der Methylaminosulfonyl-, der Ethylaminosulfonyl-, der Propylaminosulfonyl- und der Butylaminosulfonylrest, bevorzugt der Methylaminosulfonyl- und der Ethylaminosulfonylrest.

Bei dem erfindungsgemäßen Verfahren werden bevorzugt Verbindungen der Formeln

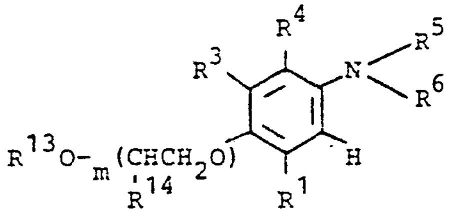

(IIa)

und

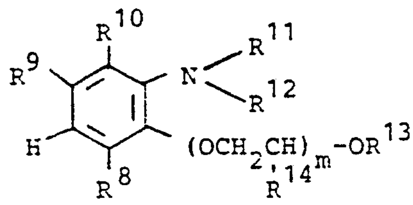

(IVa)

eingesetzt, wobei

$R^1$, $R^3$, $R^4$, $R^8$, $R^9$ und $R^{10}$ gleich oder verschieden sind und für 1 bis 3 Wasserstoffatome, 1 bis 2 Halogenatome, 1 bis 2 Alkylreste oder 1 bis 2 Alkoxyreste stehen und die Reste

$R^5$, $R^6$, $R^{11}$, $R^{12}$, $R^{13}$ und $R^{14}$ sowie m die Bedeutung der entsprechenden Reste der vorstehend aufgeführten Formeln (I-IV) haben.

Bevorzugt werden folgende am Stickstoff geschützten Aminobenzole in das erfindungsgemäße Verfahren eingesetzt :

N-(4-Bromphenyl)-acetamid, N-(4-Chlor-6-trifluormethylphenyl)-acetamid, N-(4-Chlor-5-trifluormethylphenyl)-acetamid, N-(5-Chlor-4-fluorphenyl)-acetamid, N-(4-Chlor-3,5-dimethylphenyl)-acetamid, N-(4-Brom-3,5-dimethyl)-acetamid, N-(4,6-Dichlor-3,5-dimethylphenyl)-acetamid, N-(4-Chlor-3,6-dimethylphenyl)-acetamid, N-(4-Methylphenyl)-benzolsulfonamid, N-(4-Methylphenyl)-p-toluolsulfonamid, N-(4-Methoxyphenyl)-acetamid, N-(4-Methoxyphenyl)-benzolsulfonamid, N-(4-Methoxyphenyl)-p-toluolsulfonamid, 4-Methoxyphenylcarbaminsäuremethylester, 4-Methoxyphenyl-carbaminsäureethylester, N-(4-Ethoxyphenyl)-acetamid, N-(4-Ethoxyphenyl)-benzolsulfonamid, N-(4-Ethoxyphenyl)-p-toluolsulfonamid, 4-Ethoxyphenyl-carbaminsäuremethylester, 4-Ethoxyphenyl-carbaminsäureethylester, N-(4-Ethoxy-6-methylphenyl)-acetamid, N-(4-Methoxy-6-methylphenyl)-acetamid, N-(4,6-Dimethoxyphenyl)-acetamid, N-(4-Acetyloxy-6-methylphenyl)-acetamid, N-[[4-[2-(2-Methoxyethoxy)]-ethoxyphenyl]]-acetamid, N-(Acetyloxyphenyl)-acetamid, 6-Chlor-benzoxazolon, 6-Chlor-2-methylbenzoxazol, 6-Methyl-benzoxazolon, 6,2-Dimethylbenzoxazol, N-(4,6-Dimethylphenyl)-acetamid, 4-Acetylaminobenzoesäure, N-(5-Acetylamino-4-methylphenyl)-acetamid, N-(4-Acetylaminophenyl)-acetamid, N-(4-Acetylamino-6-methylphenyl)-acetamid, N-(6-Brom-4-methylphenyl)-acetamid, N-(4-Chlorphenyl)-acetamid, N-(4,6-Dichlorphenyl)-acetamid, N-(4,5-Dichlorphenyl)-acetamid, N-(4-Brom-5-methylphenyl)-acetamid, N-(4,6-Dichlor-5-methylphenyl)-acetamid, N-(5-Fluor-4-methylphenyl)-acetamid, N-(5-Chlor-4-methylphenyl)-acetamid, N-(4,5-Dimethylphenyl)-acetamid, N-(4-Chlor-5,6-dimethylphenyl)-acetamid, N-(5-Brom-4,6-dimethylphenyl)-acetamid, N-(4-Ethylphenyl)-acetamid, N-(4-tert.-Butylphenyl)-acetamid, N-(4-Ethyl-6-methylphenyl)-acetamid, N-(5-Chlorphenyl)-acetamid, N-(5-Methylphenyl)-acetamid, N-(5-Methoxyphenyl)-acetamid, N-(5-Fluorphenyl)-acetamid, N-(3,4,6-Trichlorphenyl)-acetamid, N-(4-Chlor-6-methylphenyl)-acetamid, N-(3,4-Dichlor-6-methoxyphenyl)-acetamid, N-(5-Chlor-4-methoxyphenyl)-acetamid, N-(4-Chlor-3,6-diethoxyphenyl)-acetamid, 6-Chlor-isatosäureanhydrid, 6-Methylisatosäureanhydrid, N-(4-Methylsulfonylphenyl)-acetamid, N-(2-Methylphenyl)-acetamid, N-(2-Methylphenyl)-benzolsulfonamid, N-(2-Methylphenyl)-p-toluolsulfonamid, 2-Methylphenyl-carbaminsäuremethylester, 2-Methylphenyl-carbaminsäureethylester, N-(2-Acetyloxyphenyl)-acetamid, 2-Methylbenzoxazol, Benzoxazolon, N-(2-Methoxyphenyl)-acetamid, N-(2-Methoxyphenyl)-benzolsulfonamid, 2-Methoxyphenyl-carbaminsäuremethylester, 2-Methoxyphenylcarbaminsäureethylester, N-(2-Methoxyphenyl)-oxamidsäure, N-(2-Etnoxyphenyl)-acetamid, 2-Ethoxyphenyl-carbaminsäuremethylester, 2-Ethoxyphenyl-carbaminsäureethylester, N-(2-Ethoxyphenyl)-benzolsulfonamid, N-[[2-[2-(2-Methoxy-ethoxy)]-ethoxyphenyl]]-acetamid, N-(5-Chlor-2-methylphenyl)-acetamid, N-(5-Chlor-2-methylphenyl)-benzolsulfonamid, N-(2-Benzolsulfonylphenyl)-acetamid, N-(2-Benzolsulfonylphenyl)-methylsulfonamid, 2-Acetylaminobenzoesäure, N-(5-Chlor-2-methoxyphenyl)-benzamid, N-(5-Chlor-2-methoxyphenyl)-acetamid, N-(2-Chlor-5-methylphenyl)-acetamid, N-(2-Chlor-5-methylphenyl)-benzamid, N-(2,5-Dichlorphenyl)-acetamid, N-(2,3-Dichlorphenyl)-acetamid, N-(2-Chlorphenyl)-acetamid, N-(2,6-Dichlorphenyl)-acetamid, N-(2-Bromphenyl)-acetamid, N-(2-Fluorphenyl)-acetamid, N-(2-Acetylaminophenyl)-acetamid, N-(5-Acetylamino-2-methylphenyl)-acetamid, N-(2-Acetyloxy-5-chlorphenyl)-acetamid, N-(2-Methansulfonylphenyl)-acetamid, N-(2-Methoxy-5-methylphenyl)-acetamid, N-(2,5-Dimethoxyphenyl)-acetamid, N-(2,5-Diethoxyphenyl)-acetamid, (2-Acetylaminophenoxy)-essigsäure, N-(2,3-Dimethylphenyl)-acetamid, N-(2,6-Dimethylphenyl)-acetamid, N-(2,5-Dimethylphenyl)-acetamid, N-(3,5-Dimethylphenyl)-acetamid, N-(2-Chlor-6-methylphenyl)-acetamid, N-(2-Chlor-5-trifluormethyl)-acetamid, N-(2-Benzolsulfonyl-5-trifluormethyl)-acetamid, N-(5-Ethansulfonyl-2-methoxyphenyl)-acetamid und Isatosäureanhydrid.

Besonders bevorzugt werden in das erfindungsgemäße Verfahren folgende Verbindungen eingesetzt:

N-(4-Methoxyphenyl)-acetamid, 4-Methoxyphenyl-carbaminsäuremethyl- und -ethylester, N-(4-Ethoxyphenyl)-acetamid, 4-Ethoxyphenyl-carbaminsäuremethyl- und -ethylester, N-[[4-[2-(2-Methoxy-ethoxy)]-ethoxyphenyl]]-acetamid, N-(4-Acetyloxyphenyl)-acetamid, N-(4,6-Dimethylphenyl)-acetamid, N-(4-Acetylaminophenyl)-acetamid, N-(4-Chlorphenyl)-acetamid, N-(4,6-Dichlorphenyl)-acetamid, N-(5-Chlorphenyl)-acetamid, N-(5-Fluorphenyl)-acetamid, N-(2-Methylphenyl)-benzolsulfonamid, 2-Methylphenylcarbaminsäuremethyl- und -ethylester, N-(2-Acetyloxyphenyl)-acetamid, 2-Methylbenzoxazol, Benzoxazolon, N-(2-Methoxyphenyl)-acetamid, N-(2-Methoxyphenyl)-benzolsulfonamid, 2-Methoxyphenylcarbaminsäuremethyl- und -ethylester, N-[[2-[2-(2-Methoxy-ethoxy)]-ethoxyphenyl]]-acetamid, N-(5-Chlor-2-methylphenyl)-acetamid, N-(5-Chlor-2-methylphenyl)-benzolsulfonamid, N-(5-Chlor-2-methoxyphenyl)-acetamid, N-(5-Chlor-2-methoxyphenyl)-benzamid, N-(2-Chlor-5-methylphenyl)-benzamid, N-(2,5-Dichlorphenyl)-acetamid, N-(2,3-Dichlorphenyl)-acetamid, N-(2-Chlorphenyl)-acetamid, N-(2-Bromphenyl)-acetamid, N-(2,6-Dichlorphenyl)-acetamid, N-(2-Acetylaminophenyl)-acetamid, N-(2-Acetyloxy-5-chlorphenyl)-acetamid, N-(2-Methansulfonylphenyl)-acetamid, N-(2-Methoxy-5-methylphenyl)-acetamid, N-(2,5-Dimethoxyphenyl)-acetamid und N-(2,5-Diethoxyphenyl)-acetamid.

Die Salpetersäure wird in das erfindungsgemäße Verfahren bevorzugt in einer Konzentration von 65 bis 100, besonders bevorzugt in einer Konzentration von 80 bis 98 Gew.-% eingesetzt.

Die Menge der bei dem erfindungsgemäßen Verfahren zur Nitrierung erforderlichen Salpetersäure ist z. B. von deren Konzentration, der Nitriertemperatur und der Art der am Stickstoff gebundenen Schutzgruppe abhängig.

Im allgemeinen werden nach dem erfindungsgemäßen Verfahren pro Mol Einsatzprodukt mindestens 1,1 Mol 100 gew.-%ige Salpetersäure, bevorzugt 1,1 bis 9 Mol, besonders bevorzugt 1,2 bis 7 Mol, 100 gew.-%ige Salpetersäure eingesetzt.

Bei Verwendung wasserentziehender Mittel, wie Phosphorpentoxid, kann gegebenenfalls auch mit molaren Mengen an Salpetersäure gearbeitet werden.

Bei Einsatz von 50 gew.-%iger Salpetersäure werden im allgemeinen mindestens 1,3 Mol Salpetersäure pro Mol Einsatzprodukt verwendet. Bevorzugt werden pro Mol Einsatzprodukt 1,3 bis 15 Mol, besonders bevorzugt 1,4 bis 12 Mol, 50 %iger Salpetersäure eingesetzt. Für Salpetersäurekonzentrationen, die zwischen 50 und 100 Gew.-% liegen, wird zweckmäßigerweise das günstigste Molverhältnis von Salpetersäure zu Ausgangsverbindung durch Vorversuche ermittelt.

Das erfindungsgemäße Verfahren wird in Gegenwart von Methylenchlorid durchgeführt. Es ist jedoch auch möglich, anstelle von Methylenchlorid andere halogenierte aliphatische Kohlenwasserstoffe mit 1 bis 6, bevorzugt 1 bis 3, Kohlenstoffatomen in das erfindungsgemäße Verfahren einzusetzen, wie Chloroform, 1,1-Dichlorethan, 1,2-Dichlorethan, 1,1,1-Trichlorethan, 1,1-Dichlorpropan, 2,2-Dichlorpropan und/oder Monofluortrichlormethan.

Die Menge an Methylenchlorid kann in weiten Bereichen variiert werden. Im allgemeinen beträgt die Menge an Methylenchlorid etwa 400 bis etwa 2 500 ml pro Mol Ausgangsverbindung. Bevorzugt werden auf 1 Mol Ausgangsverbindung 450 bis 2 000 ml, besonders bevorzugt 500 bis 1 500 ml, Methylenchlorid eingesetzt. Werden andere halogenierte aliphatische Kohlenwasserstoffe eingesetzt, so können diese in der gleichen Menge verwendet werden.

Das erfindungsgemäße Verfahren wird vorzugsweise in Gegenwart von salpetriger Säure und/oder Salpetrigsäure bildenden Substanzen durchgeführt.

Als Salpetrigsäure bildende Substanzen können bei der erfindungsgemäßen Nitrierung die Alkali- und/oder Erdalkalisalze der salpetrigen Säure wie Natrium-, Kalium- oder Bariumnitrit und/oder das Anhydrid der salpetrigen Säure und/oder Nitrose Gase, wie Stickstoffmonoxid, Distickstofftrioxid und Distickstofftetroxid, und/oder Nitrosylschwefelsäure zugesetzt werden. Bevorzugt werden die Alkalisalze der salpetrigen Säure, besonders bevorzugt Natriumnitrit zugesetzt.

Die Menge der gegebenenfalls zugesetzten salpetrigen Säure und/oder Salpetrigsäure bildenden Substanzen beträgt im allgemeinen 0,01 bis 5 Mol-%, bevorzugt 0,03 bis 4 Mol-%, besonders bevorzugt 0,05 bis 3 Mol-%, bezogen auf eingesetztes Ausgangsprodukt.

Als wasserbindendes Mittel wird bevorzugt Phosphorpentoxid dem Reaktionsgemisch zugesetzt. Im allgemeinen werden etwa 0,25 bis 1 Mol, bevorzugt 0,3 bis 0,5 Mol, besonders bevorzugt 0,32 bis 0,36 Mol, Phosphorpentoxid pro Mol eingesetztes Ausgangsprodukt zugesetzt.

Das erfindungsgemäße Verfahren wird üblicherweise bei Temperaturen von etwa 0 bis etwa 80 °C,

bevorzugt bei 5 bis 50 °C, besonders bevorzugt bei 10 bis 40 °C, durchgeführt. In einer ganz besonders bevorzugten Arbeitsweise wird die Reaktion unter Rückflußbedingungen und Normaldruck durchgeführt.

Das erfindungsgemäße Verfahren kann in dem angegebenen Temperaturbereich sowohl bei Normaldruck als auch bei vermindertem oder erhöhtem Druck, beispielsweise bei Drücken im Bereich von etwa 0,2 bis etwa 3,5 bar, bevorzugt bei 0,25 bis 1,4 bar, besonders bevorzugt bei 0,3 bis 1 bar, durchgeführt werden.

Die Reaktionszeit der Nitrierung hängt bei konstanter Temperatur im wesentlichen von der Salpetersäurekonzentration und der Menge der eingesetzten Salpetersäure ab. Die Reaktionszeit ist umso kürzer, je höher konzentriert die Salpetersäure und je größer der Überschuß an Salpetersäure, bezogen auf das Ausgangsprodukt, ist. Im allgemeinen betragen die Reaktionszeiten etwa 15 Minuten bis etwa 10 Stunden.

Das erfindungsgemäße Verfahren wird im allgemeinen in der Weise durchgeführt, daß man das Einsatzprodukt und die Salpetersäure synchron im zuvor beschriebenen Temperaturbereich in vorgelegtes Methylenchlorid, das gegebenenfalls einen Teil der zur Nitrierung erforderlichen Salpetersäure und gegebenenfalls noch Zusätze wie salpetrige Säure und/oder deren Salze und/oder deren Anhydrid und/oder Nitrose Gase und/oder Nitrosylschwefelsäure und gegebenenfalls noch ein wasserentziehendes Mittel, wie Phosphorpentoxid, enthält, eindosiert. Dabei ist es im allgemeinen nicht erforderlich, das Reaktionsgemisch zu kühlen, da die Reaktionswärme durch das siedende Methylenchlorid abgeführt werden kann. Das Einsatzprodukt kann dabei gelöst oder suspendiert in Methylenchlorid eingesetzt werden. Es ist selbstverständlich auch möglich, das Einsatzprodukt in fester Form oder als Schmelze in das erfindungsgemäße Verfahren einzusetzen. Bei basischen Einsatzprodukten ist es ebenfalls möglich, diese als salpetersaure Salze, welche durch Zusatz von etwa 98 %iger Salpetersäure, beispielsweise in Methylenchlorid, erhalten werden können, einzusetzen.

Bei Verwendung von höherkonzentrierter Salpetersäure kann es vorteilhaft sein, eine Mischung von Salpetersäure und Methylenchlorid synchron mit dem Einsatzprodukt zuzudosieren. In diesem Fall wird die Qualität an erwünschtem Nitroaminobenzol noch verbessert.

Die Menge der mit dem Methylenchlorid vorgelegten Salpetersäure kann in weiten Bereich schwanken. Im allgemeinen beträgt die Menge an vorgelegter Salpetersäure etwa 1 bis 70 Gew.-%, bevorzugt 2 bis 50 Gew.-%, besonders bevorzugt 5 bis 40 Gew.-%, der zur Nitrierung erforderlichen Gesamtmenge.

Die oben erwähnten Zusätze an salpetriger Säure und Salpetrigsäure bildenden Substanzen können auch der einzudosierenden Salpetersäure oder gleichzeitig mit dem Einsatzprodukt und der Salpetersäure zugegeben werden. Die Geschwindigkeit der Zugabe von Einsatzprodukt und Salpetersäure richtet sich nach der abzuführenden Reaktionswärme und beträgt bei einem diskontinuierlichen Verfahren je nach Ansatzgröße gewöhnlich etwa 0,5 bis 10 Stunden, bevorzugt 2 bis 5 Stunden.

Es ist natürlich auch möglich, daß man das Einsatzprodukt gelöst oder suspendiert in Methylenchlorid vorlegt und mit Salpetersäure, die gegebenenfalls mit Methylenchlorid vermischt ist, versetzt. Diese Arbeitsweise ist jedoch gegenüber den zuvor beschriebenen Arbeitsweisen weniger bevorzugt.

Neben den zuvor beschriebenen diskontinuierlichen Fahrweisen ist auch eine kontinuierliche Durchführung des erfindungsgemäßen Verfahrens möglich. Dabei wird vorteilhafterweise unter Rückführung oder Kreisführung von teilweise oder vollständig ausreagiertem Nitriergemisch, beispielsweise in einem Schlaufenreaktor, gearbeitet.

Die Aufarbeitung des Nitriergemisches kann in der Weise erfolgen, daß das Reaktionsgemisch in eine wäßrige, etwa 1 bis 25 gew.-%ige (Angaben in Gew.-% beziehen sich auf den Gehalt an freier Base nach Neutralisation der überschüssigen Salpetersäure), bevorzugt in eine 5 bis 20 gew.-%ige, Lösung oder Suspension eines Ammonium-, Alkali- und/oder Erdalkalihydroxids und/oder Carbonats, bevorzugt in eine wäßrige Alkalihydroxidlösung, besonders bevorzugt in eine wäßrige Natronlauge, gegeben wird. Dabei wird die Temperatur vorzugsweise so gewählt (z. B. 42 bis 70 °C), daß das Methylenchlorid, gegebenenfalls zusammen mit Wasser, abdestilliert. Die alkalische Abspaltung der Schutzgruppe (die alkalische Abspaltung der Schutzgruppe kommt nicht in Frage für Aminobenzole, deren Aminogruppe durch einen Alkylsulfonyl- oder einen Arylsulfonylrest geschützt ist) wird vorzugsweise bei etwa 70 bis 100 °C, insbesondere bei 75 bis 95 °C, durchgeführt bzw. zu Ende geführt. In einigen Fällen kann es auch vorteilhaft sein, z. B. bei den Harnstoffen, die alkalische Spaltung unter Druck im Temperaturbereich von 100 bis 150 °C, bevorzugt bei 100 bis 130 °C, vorzunehmen.

Nach der Spaltung wird das Reaktionsprodukt bei etwa 0 bis 85 °C, bevorzugt bei 40 bis 80 °C, durch z. B. Filtration oder Phasentrennung in üblicher Weise isoliert.

Man kann die alkalische Abspaltung der Schutzgruppen statt im wäßrigen System auch in einem wäßrig/alkoholischen — oder in einem alkoholischen System durchführen.

Als geeignete Alkohole kommen hierfür vorzugsweise aliphatische Alkohole mit 1 bis 4 Kohlenstoffatomen in Betracht, welche bei oder nach erfolgter Abspaltung der Schutzgruppe zusammen mit Wasser abdestilliert werden können. Bevorzugt wird für die Spaltung Methanol, Ethanol, n-Propanol, Isopropanol und tert.-Butanol, besonders bevorzugt Methanol, eingesetzt.

Statt das Reaktionsgemisch in eine alkalisch wäßrige, wäßrig/alkoholische oder alkoholische Lösung oder Suspension zu geben, kann es vorteilhaft sein, die im Reaktionsgemisch enthaltene überschüssige Salpetersäure zunächst ganz oder teilweise, bevorzugt vollständig, mit einer Base, bevorzugt mit

Ammoniak, Ammonium- oder Alkalihydroxid, besonders bevorzugt mit Natronlauge, zu neutralisieren, das Methylenchlorid in Gegenwart von Wasser abzudestillieren und die zurückbleibende wäßrige Suspension oder Emulsion, gegebenenfalls nach Zwischenisolierung der geschützten Nitroaminobenzole, zur Schutzgruppenabspaltung mit einer Base, bevorzugt mit Natronlauge, gegebenenfalls unter Zusatz eines Alkohols, zu versetzen und wie zuvor beschrieben aufzuarbeiten.

Nach dem erfindungsgemäßen Verfahren erfolgt die Aufarbeitung des Nitriergemisches bevorzugt in der Weise, daß man die überschüssige Salpetersäure ganz oder teilweise aus dem Nitriergemisch, gegebenenfalls nach Versetzen mit Wasser, durch Phasentrennung und/oder durch Extraktion mit Wasser entfernt, wobei man gegebenenfalls die Salpetersäure vorher neutralisiert.

Bevorzugt wird die überschüssige Salpetersäure mit möglichst wenig Wasser diskontinuierlich oder kontinuierlich zu mehr als 95 %, bevorzugt mehr als 98 % extrahiert. Auf diese Weise erhält man eine wäßrige Salpetersäurelösung, aus der eine Salpetersäure von etwa 55 bis 68 Gew.-% nach bekannten technischen Verfahren, z. B. durch Distillation, zurückgewonnen werden kann.

Weiterhin ist es möglich, die überschüssige Salpetersäure nur teilweise, beispielsweise zu etwa 50 bis 95 %, durch Phasentrennung, gegebenenfalls nach Versetzen mit Wasser, und/oder durch Extraktion mit Wasser zu entfernen. Diese Verfahrensvariante hat neben einem geringen technischen Aufwand besonders bei in Methylenchlorid schwerlöslichen, am Stickstoff geschützten Nitroaminobenzolen den Vorteil, daß die Löslichkeit der nitrierten Ausgangsverbindungen in Methylenchlorid durch die verbleibende Salpetersäure gesteigert wird.

Zur Entfernung der überschüssigen Salpetersäure aus dem Nitriergemisch kann es weiterhin vorteilhaft sein, die Salpetersäure zunächst mit Ammoniak oder einer wäßrigen Lösung eines Ammonium- oder Alkalihydroxids, bevorzugt mit einer wäßrigen Alkalihydroxidlösung, besonders bevorzugt mit wäßrigem Natriumhydroxid, teilweise oder vollständig zu neutralisieren und das gebildete Nitrat, gegebenenfalls nach Versetzen mit Wasser, durch eine anschließende Phasentrennung abzutrennen.

Die weitere Aufarbeitung des extrahierten Reaktionsgemisches kann nach dem erfindungsgemäßen Verfahren so erfolgen, daß man die Schutzgruppe der nitrierten Aminobenzole, gegebenenfalls nach vorheriger Abtrennung des Methylenchlorids, sauer oder alkalisch abspaltet.

Gemäß einer bevorzugten Arbeitsweise wird die Aminoschutzgruppe in salzsaurer oder schwefelsaurer Lösung, besonders bevorzugt in schwefelsaurer Lösung, abgespalten. Hierbei wird vorzugsweise so verfahren, daß man das nach der Extraktion vorliegende Produktgemisch zur Abspaltung der Aminoschutzgruppe in eine wäßrige, schwefelsaure Lösung gibt, deren Konzentration in Abhängigkeit von der abzuspaltenden Schutzgruppe in an sich bekannter Weise ausgewählt wird. Im allgemeinen beträgt die Konzentration der Schwefelsäure etwa 1 bis 85 Gew.-%, bevorzugt 5 bis 80 Gew.-%.

Die Temperatur der vorgelegten wäßrigen Schwefelsäure wird dabei vorzugsweise so gewählt, daß das Methylenchlorid zusammen mit Wasser abdestilliert. Im allgemeinen betragen die Temperaturen etwa 45 bis 70 °C.

Die Temperaturen, bei denen die Schutzgruppe der Aminoverbindungen üblicherweise abgespalten werden, betragen etwa 60 bis 100 °C, bevorzugt 70 bis 95 °C.

Falls die überschüssige Salpetersäure nicht vollständig aus dem Methylenchlorid entfernt worden ist, kann es vorteilhaft sein, der vorgelegten wäßrigen Schwefelsäure Substanzen zuzusetzen, welche in der Lage sind, salpetrige Säure zu binden oder zu zerstören. Zum Beispiel kann man Harnstoff und/oder Amidosulfonsäure zusetzen. Bevorzugt wird pro Mol eingesetztem Ausgangsprodukt etwa 0,005 bis etwa 0,15 Mol Harnstoff und/oder Amidosulfonsäure zugegeben.

Nachdem die Schutzgruppe abgespalten worden ist, wird das Reaktionsprodukt, gegebenenfalls nach Klärung der das Reaktionsprodukt enthaltenden sauren Lösung mit Aktivkohle und/oder gegebenenfalls nach Verdünnung der sauren Lösung oder Suspension mit Wasser und/oder gegebenenfalls nach Pufferung der sauren Lösung oder Suspension mit Ammoniak oder einer wäßrigen Lösung eines Ammonium- oder Alkalihydroxids, bevorzugt mit einer wäßrigen Alkalihydroxidlösung, besonders bevorzugt mit wäßrigem Natriumhydroxid, bei etwa 0 bis 60 °C, bevorzugt bei 15 bis 45 °C, durch Filtration oder Phasentrennung in üblicher Weise isoliert.

Statt die saure Abspaltung der Aminoschutzgruppe in einem wäßrigen System vorzunehmen, kann es auch hier vorteilhaft sein, die Abspaltung, wie zuvor beschrieben, in einem wäßrig/alkoholischen oder in einem alkoholischen System durchzuführen.

Erfolgt die Abspaltung der Schutzgruppe in alkalischem Medium (die alkalische Abspaltung der Schutzgruppe kommt nicht in Frage für Aminobenzole, deren Aminogruppe durch einen Alkylsulfonyl- oder Arylsulfonylrest geschützt ist), so wird beispielsweise in der Weise verfahren, daß das von der überschüssigen Salpetersäure durch Extraktion vollständig oder teilweise befreite Methylenchlorid, welches das nitrierte Ausgangsprodukt suspendiert oder bevorzugt gelöst enthält, in eine wäßrige, wäßrig/alkoholische oder alkoholische, etwa 1 bis 25 gew.-%ige, bevorzugt 5 bis 20 gew.-%ige (die Angaben in Gew.-% beziehen sich auf den Gehalt an freier Base nach Neutralisation der überschüssigen Salpetersäure), Lösung oder Suspension eines Ammonium-, Alkali- und/oder Erdalkalihydroxids und/oder Carbonats, bevorzugt in eine wäßrige Alkalihydroxidlösung, besonders bevorzugt in eine wäßrige Natriumhydroxidlösung, gegeben und, wie zuvor beschrieben, weiter aufgearbeitet wird.

In einigen Fällen, wie bei der Nitrierung von 2-Acetylaminoanisol, die nach dem erfindungsgemäßen Verfahren zwar eine hohe para-Selektivität ergibt, deren meta-Nitrierung jedoch nicht vollständig

unterdrückt werden kann, kann es vorteilhaft sein, das erhaltene 5-Nitro- und 4-Nitro-2-acetylaminoanisol-Gemisch in alkalischem Medium, wie zuvor beschrieben, zu spalten. Bei dieser Arbeitsweise erhält man höhere Ausbeuten an 5-Nitro-2-aminoanisol und farblosere Produkte als bei der sauren Spaltung. Wird ein noch reineres 5-Nitro-2-aminoanisol gewünscht, so läßt sich dieses durch eine sich anschließende Spaltung in saurem Medium herstellen.

Bei dem erfindungsgemäßen Verfahren ist es selbstverständlich auch möglich, das Reaktionsgemisch nach beendeter Nitrierung, ohne vorherige Entfernung der überschüssigen Salpetersäure oder nach vollständiger oder teilweiser Neutralisation der überschüssigen Salpetersäure, auf Wasser zu geben, anschließend das Methylenchlorid in an sich bekannter Weise zu entfernen und das geschützte Nitroaminobenzol aus der wäßrigen Suspension oder Emulsion durch Filtration oder Phasentrennung zu isolieren.

Bevorzugt wird das Reaktionsgemisch nach beendeter Nitrierung und anschließender vollständiger oder teilweiser Entfernung der überschüssigen Salpetersäure durch Extraktion mit Wasser auf Wasser gegeben, gegebenenfalls nach vorheriger Neutralisation der Salpetersäure oder nach Phasentrennung. Anschließend wird wie im Abschnitt zuvor geschildert, aufgearbeitet.

Falls es erforderlich sein sollte, können die erhaltenen Nitroaminobenzole noch weiter entweder durch Umkristallisation, Sublimation oder Destillation gereinigt werden.

Gegenüber den Verfahren nach dem Stand der Technik bietet das erfindungsgemäße Verfahren wesentliche Vorteile. So wird bei dem erfindungsgemäßen Verfahren eine größere Ausbeute und eine höhere Produktqualität (Reinheit) erzielt.

Weiterhin wird bei dem erfindungsgemäßen Verfahren auf die Verwendung von zusätzlichen Mineralsäuren, wie Schwefelsäure, als Lösungs- oder Verdünnungsmittel vollständig verzichtet. Dies bedingt gegenüber dem Stand der Technik nicht nur eine kostenmindernde Einsparung an Mineralsäure, sondern löst gleichzeitig die Probleme, die sonst mit der Aufarbeitung oder Beseitigung derartiger Säuren verbunden sind. Außerdem ist bei dem erfindungsgemäßen Verfahren die Rückgewinnung der überschüssigen Salpetersäure und des eingesetzten Methylenchlorids einfach und damit äußerst wirtschaftlich durchzuführen.

Ein weiterer Vorteil des erfindungsgemäßen Verfahrens ist darin zu sehen, daß die Nitrierung auch bei größeren Ansätzen sicherheitstechnisch ohne Komplikation durchgeführt werden kann. Es ist ferner überraschend, daß gegenüber den bisher bekannten Lösungsmittel-Nitrierverfahren bei dem erfindungsgemäßen Verfahren die Ausbeuten und Qualitäten der isolierten Nitroaminobenzole bzw. der gegebenenfalls zwischenisolierten am Stickstoff geschützten Nitroaminobenzole nicht mit zunehmender Dosierdauer der Einsatzprodukte, wie es bei größeren Ansätzen wegen der Abführung der entstehenden Reaktionswärme erforderlich wird, abnehmen.

Es ist außerdem sehr überraschend, daß bei dem erfindungsgemäßen Verfahren keine Komplikationen durch die Bildung von Diazoniumsalzen auftraten. Dies würde nämlich, wie in der DE-AS 2 226 405 beschrieben, wegen der Gefährlichkeit des festen Diazoniumnitrats eine technische Durchführung des erfindungsgemäßen Verfahrens praktisch ausschließen.

Die Nitroaminobenzole sind wichtige Zwischenprodukte zur Herstellung von Farbstoffen Pflanzenschutzmitteln, Pharmazeutika, Kunststoffen und Sprengstoffen (siehe Ullmanns Enzyklopädie der technischen Chemie, 4. Auflage, Band 17, Seite 383 (1979)) und können außerdem noch in der Pelz- und Haarfärberei eingesetzt werden (s. Ullmanns Enzyklopädie der technischen Chemie, 3. Auflage, Band 12, Seite 767 (1960)).

Die nachfolgenden Beispiele sollen das erfindungsgemäße Verfahren erläutern, ohne es jedoch auf diese Beispiele einzuschränken.

## Beispiel 1

In einem 4 l Vierhalskolben, ausgerüstet mit Rührer, Rückflußkühler, Thermometer und zwei Tropftrichtern, von welchen einer beheizbar ist, werden 2 600 ml Methylenchlorid, 25,7 g 98 %ige (0,4 Mol) Salpetersäure und 9,2 g 30 %ige (0,04 Mol) wäßrige Natriumnitritlösung vorgelegt, unter Normaldruck zum Sieden erhitzt und innerhalb von 3 Stunden werden 231,4 g 98 %ige (3,6 Mol) Salpetersäure und 332 g (2 Mol) 99,5 %iges technisches 2-Acetylaminoanisol als Schmelze simultan zugetropft. Nach beendetem Eintropfen wird noch 30 Minuten unter Rückfluß erhitzt und die erhaltene Lösung mit 200 ml Wasser versetzt. Bei 20 °C werden die Phasen getrennt und anschließend läßt man unter Rühren die Methylenchloridphase bei ca. 50 bis 60 °C in verdünnte Schwefelsäure, bestehend aus 1 764 ml Wasser und 196 g 100 %ige (2 Mol) Schwefelsäure, welche noch zusätzlich 9,7 g (0,1 Mol) Amidosulfonsäure enthält, einfließen, wobei Methylenchlorid mit etwas Wasser abdestilliert. Anschließend wird die Temperatur bis auf 95 °C gesteigert und 2 Stunden gehalten, wobei die Acetylaminogruppe zur Aminogruppe hydrolysiert. Danach wird mit 1 960 ml Wasser versetzt, auf 40 °C abgekühlt, 45 Minuten bei 40 °C nachgerührt, abgesaugt und mit Wasser säurefrei gewaschen. Das Feuchtprodukt wird bis zur Gewichtskonstanz im Vakuum bei 60 bis 70 °C getrocknet.

Ausbeute : Produkt trocken 272,6 g (80,6 % der Theorie) ;
Reinheit : 99,4 %iges 5-Nitro-2-aminoanisol.

Beispiel 2

Es wurde wie in Beispiel 1 verfahren, jedoch wurden 1 200 ml Methylenchlorid und 23,1 g 98 %ige (0,36 Mol) Salpetersäure mit dem Natriumnitrit vorgelegt, anschließend 208,3 g 98 %ige (3,24 Mol) Salpetersäure simultan mit der Schmelze dosiert und die Phasentrennung bei 30 bis 35 °C durchgeführt.
Ausbeute : Produkt trocken 270,5 g (79,6 % der Theorie) ;
Reinheit : 99,0 %iges 5-Nitro-2-aminoanisol.

Beispiel 3

Es wurde wie in Beispiel 1 verfahren, jedoch wurden 1 900 ml Methylenchlorid und 59,7 g 67,5 %ige (0,64 Mol) Salpetersäure mit dem Natriumnitrit bei 20 °C vorgelegt, anschließend 537,3 g 67,5 %ige (5,76 Mol) Salpetersäure simultan mit der Schmelze dosiert, wobei die Reaktionstemperatur durch externe Kühlung bei 20 °C gehalten wurde. Nach beendetem Eintropfen wurde noch 30 Minuten bei 20 °C nachgerührt und die erhaltene Lösung zur Phasentrennung bei 20 °C mit 6 ml Wasser versetzt.
Ausbeute : Produkt trocken 268,0 g (79,1 % der Theorie) ;
Reinheit : 99,3 %iges 5-Nitro-2-aminoanisol.

Beispiel 4

In einem 4 l Vierhalskolben, ausgerüstet mit Rührer, Rückflußkühler, Thermometer und zwei Tropftrichtern, von welchen einer beheizbar ist, werden 1 800 ml Methylenchlorid, 23,1 g 98 %ige (0,36 Mol) Salpetersäure und 9,2 g 30 %ige (0,04 Mol) wäßrige Natriumnitritlösung vorgelegt, unter Normaldruck zum Sieden erhitzt und innerhalb von 3 Stunden werden 208,3 g 98 %ige (3,24 Mol) Salpetersäure und 332 g (2 mol) 99,5 %iges technisches 2-Acetylaminoanisol als Schmelze simultan zugetropft. Nach beendetem Eintropfen wird noch 30 Minuten unter Rückfluß erhitzt und die erhaltene Lösung mit 200 ml Wasser versetzt. Bei 30 bis 35 °C werden die Phasen getrennt und anschließend wird die untere Methylenchloridphase noch 2-mal mit je 100 ml 30 bis 35 °C warmem Wasser bei gleicher Temperatur extrahiert. Nach beendeter Extraktion wird die Methylenchloridphase wie in Beispiel 1 beschrieben, weiter aufgearbeitet.
Ausbeute : Produkt trocken 270,3 g (80,1 % der Theorie) ;
Reinheit : 99,7 %iges 5-Nitro-3-aminoanisol.

Beispiel 5

Es wurde wie in Beispiel 1 verfahren, jedoch wurden statt 2 600 ml, 1 800 ml Methylenchlorid vorgelegt. Nach beendetem Eintropfen wird noch 30 Minuten unter Rückfluß erhitzt, die erhaltene Lösung unter Rückfluß mit einer 45 %igen wäßrigen Natronlauge auf pH 5 gestellt und das Reaktionsgemisch mit 300 ml Wasser versetzt. Bei 30 bis 35 °C werden die Phasen getrennt und anschließend läßt man unter Rühren die Methylenchloridphase bei ca. 50 bis 60 °C in verdünnte Schwefelsäure, bestehend aus 784 ml Wasser, 196 g 100 %iger (2 Mol) Schwefelsäure, welche noch zusätzlich 9,7 g (0,1 Mol) Amidosulfonsäure enthält, einfließen, wobei Methylenchlorid mit etwas Wasser abdestilliert. Anschließend wird die Temperatur bis auf 95 °C gesteigert und 2 Stunden gehalten, wobei die Acetylaminogruppe zur Aminogruppe hydrolysiert. Danach wird mit 2 940 ml Wasser versetzt, auf 40 °C abgekühlt, 45 Minuten bei 40 °C nachgerührt, abgesaugt und mit Wasser säurefrei gewaschen. Das Feuchtprodukt wird bis zur Gewichtskonstanz im Vakuum bei 60 bis 70 °C getrocknet.
Ausbeute : Produkt trocken 273,9 g (80,9 % der Theorie) ;
Reinheit : 99,3 %iges 5-Nitro-2-aminoanisol.

Beispiel 6

Es wurde wie im Beispiel 5 verfahren, jedoch wurde der verdünnten Schwefelsäure keine Amidosulfonsäure zugesetzt. Die Ausbeute und Reinheit des Produktes war praktisch die gleiche, wie bei dem Beispiel 5.

Beispiel 7

In einem 2 l Vierhalskolben, ausgerüstet mit Rührer, Rückflußkühler, Thermometer und zwei Tropftrichtern, von welchen einer beheizbar ist, werden 1 200 ml Methylenchlorid, 34,7 g 80 %ige (0,44 Mol) Salpetersäure und 9,2 g 30 %ige (0,04 Mol) wäßrige Natriumnitritlösung vorgelegt, unter Normaldruck zum Sieden erhitzt und innerhalb von 3 Stunden werden 311,8 g 80 %ige (3,96 Mol) Salpetersäure und 332,3 g (2 Mol) 99,4 %iges technisches 2-Acetylaminoanisol als Schmelze simultan zugetropft. Nach beendetem Eintropfen wird noch 30 Minuten unter Rückfluß erhitzt, die erhaltene Lösung mit 200 ml Wasser versetzt, dieses Reaktionsgemisch auf vorgelegte 731 ml Wasser gegeben, anschließend mit einer 45 %igen wäßrigen Natronlauge bei ca. 20 °C auf pH 5 gestellt. Danach wird das

Methylenchlorid zunächst bei einer Sumpftemperatur von ca. 50 °C, welche am Ende bis auf 95 °C gesteigert wird, destillativ entfernt, auf 40 °C abgekühlt, 30 Minuten bei 40 °C nachgerührt, abgesaugt und 2-mal mit Wasser nachgewaschen. Das Feuchtprodukt wird bis zur Gewichtskonstanz im Vakuum bei 60 bis 70 °C getrocknet.

Ausbeute : Produkt trocken 413,2 g Rohprodukt.

Dieses Rohprodukt wird in verdünnte Schwefelsäure, bestehend aus 784 ml Wasser und 196 g 100 %ige (2 Mol) Schwefelsäure, eingetragen, auf 95 °C erhitzt und diese Temperatur 3 Stunden gehalten, wobei die Acetylaminogruppe zur Aminogruppe hydrolysiert. Danach wird wie im Beispiel 5 beschrieben weiterverfahren.

Ausbeute : Produkt trocken 278,3 g (82,1 % der Theorie) ;
Reinheit : 99,2 %iges 5-Nitro-2-aminoanisol.

Beispiel 8

In einem 4 l Vierhalskolben, ausgerüstet mit Rührer, Rückflußkühler, Thermometer und zwei Tropftrichtern, von welchen einer beheizbar ist, werden 1 400 ml Methylenchlorid, 23,1 g 98 %ige (0,36 Mol) Salpetersäure und 9,2 g 30 %ige (0,04 Mol) wäßrige Natriumnitritlösung vorgelegt, unter Normaldruck zum Sieden erhitzt und innerhalb von 3 Stunden werden 208,3 g 98 %ige (3,24 Mol) Salpetersäure und 332,3 g (2 Mol) 99,4 %iges technisches 2-Acetylaminoanisol als Schmelze simultan zugetropft. Nach beendetem Eintropfen wird noch 30 Minuten unter Rückfluß erhitzt, die erhaltene Lösung mit einer Anschlämmung von 20,5 g Bleicherde in 200 ml Wasser versetzt, anschließend noch 30 Minuten unter Rückfluß erhitzt, heiß abfiltriert, der Filterrückstand noch einmal mit ca. 50 ml Methylenchlorid nachgewaschen und die Phasen bei 30 bis 35 °C getrennt. Die Methylenchloridphase läßt man unter Rühren in 1 000 ml Wasser einlaufen und anschließend wird mit einer 45 %igen wäßrigen Natronlauge bei ca. 20 bis 30 °C auf pH 5 gestellt. Danach wird wie in Beispiel 7 beschrieben weiterverfahren.

Ausbeute : Produkt trocken 401,5 g Rohprodukt ;
Ausbeute : Produkt trocken 271,5 g (80,5 % der Theorie) ;
Reinheit : 99,7 %iges 5-Nitro-2-aminoanisol.

Beispiel 9

Es wurde wie in Beispiel 2 verfahren, jedoch wird nach der Phasentrennung bei 30 bis 35 °C die Methylenchloridphase unter Rühren bei ca. 45 °C in verdünnte Natronlauge, bestehend aus 2 280 ml Wasser und 145,6 g (3,64 Mol) NaOH einfließen gelassen, wobei Methylenchlorid mit etwas Wasser abdestilliert. Anschließend wird die Temperatur bis auf 80 °C gesteigert, der Rest Methylenchlorid bei vermindertem Druck entfernt und die Temperatur von 80 °C 2 Stunden gehalten, wobei die Acetylaminogruppe des 5-Nitro-2-acetylaminoanisols vollständig zur Aminogruppe hydrolysiert. Es wird bei 80 °C abgesaugt und mit Wasser alkalifrei gewaschen. Das Feuchtprodukt wird bis zur Gewichtskonstanz im Vakuum bei 60 bis 70 °C getrocknet.

Ausbeute : Produkt trocken : 314,6 g (83,1 % der Theorie).
Reinheit : 88,8 %iges 5-Nitro-2-aminoanisol, welches noch
ca. 8,5 % 4-Nitro-2-acetylaminoanisol und
ca. 2,5 % 4-Nitro-2-aminoanisol enthält.

Beispiel 10

Es wurde wie im Beispiel 9 verfahren, jedoch wird das Feuchtprodukt, nachdem es mit Wasser alkalifrei gewaschen ist, noch einmal mit ca. 800 ml 5 gew.-%iger Schwefelsäure auf der Nutsche suspendiert, abgesaugt und mit Wasser säurefrei gewaschen. Das Feuchtprodukt wird bis zur Gewichtskonstanz im Vakuum bei 60 bis 70 °C getrocknet.

Ausbeute : Produkt trocken 299,2 g (82,6 % der Theorie).
Reinheit : 92,8 %iges 5-Nitro-2-aminoanisol, welches noch
ca. 7 % 4-Nitro-2-acetylaminoanisol und
ca. 0,1 % 4-Nitro-2-aminoanisol enthält.

Beispiel 11

Es wurde wie im Beispiel 9 verfahren, jedoch wurde das erhaltene Produkt anschließend bei ca. 60 °C in verdünnte Schwefelsäure, bestehend aus 2 979 ml Wasser und 156,8 g 100 %ige (1,6 Mol) Schwefelsäure eingetragen, auf 95 °C erhitzt und die Temperatur 2,5 Stunden gehalten. Anschließend wird auf 40 °C abgekühlt, 45 Minuten bei 40 °C nachgerührt, abgesaugt und mit Wasser säurefrei gewaschen. Das Feuchtprodukt wird bis zur Gewichtskonstanz im Vakuum bei 60 bis 70 °C getrocknet.

Ausbeute : Produkt trocken 271,7 g (80,7 % der Theorie).
Reinheit : 99,9 %iges 5-Nitro-2-aminoanisol.

## Beispiel 12

In einem 2 l Vierhalskolben, ausgerüstet mit Rührer, Rückflußkühler, Thermometer und zwei Tropftrichtern, von welchen einer beheizbar ist, wurden 1 200 ml Methylenchlorid, 7,7 g 98 %ige (0,12 Mol) Salpetersäure und 4,6 g 30 %ige (0,02 Mol) wäßrige Natriumnitritlösung vorgelegt, unter Normaldruck zum Sieden erhitzt und innerhalb von 3 Stunden 146,6 g 98 %ige (2,28 Mol) Salpetersäure und 334,1 g (2 Mol) 98,9 %iges technisches 4-Acetylaminoanisol als Schmelze simultan zugetropft. Nach beendetem Eintropfen wird noch 30 Minuten unter Rückfluß erhitzt und die erhaltene Lösung läßt man unter Rühren bei 50 bis 60 °C in verdünnte Natronlauge, bestehend aus 792 ml Wasser und 104 g (2,6 Mol) NaOH einfließen, wobei Methylenchlorid mit etwas Wasser abdestilliert. Anschließend wird die Temperatur bis auf 95 °C gesteigert und 45 Minuten gehalten, wobei die Acetylaminogruppe des 3-Nitro-4-acetylaminoanisols vollständig zur Aminogruppe hydrolysiert. Es wird auf 50 °C abgekühlt, 1 Stunde bei 50 °C nachgerührt, abgesaugt, mit Wasser alkalifrei gewaschen und noch ca. 0,5 Stunden gut trocken gesaugt.

Ausbeute : Produkt feucht 356,5 g (96,1 % der Theorie) ;
Gehalt : 90,7 %iges 3-Nitro-4-aminoanisol.

Nach dem Trocknen des Feuchtproduktes bis zur Gewichtskonstanz im Vakuum bei 60 bis 70 °C weist das Produkt eine Reinheit von 99,1 % auf.

## Beispiel 13

Es wurde wie in Beispiel 12 verfahren, jedoch wird nach beendetem Eintropfen und 30-minütigem Nachrühren unter Rückfluß die erhaltene Lösung mit 200 ml Wasser versetzt. Bei 30 bis 35 °C wurden die Phasen getrennt. Die untere Methylenchloridphase wird unter Rühren bei 50 bis 60 °C in verdünnte Natronlauge, bestehend aus 1 672 ml Wasser und 92 g (2,3 Mol) NaOH einfließen gelassen, wobei Methylenchlorid mit etwas Wasser abdestilliert. Anschließend wird die Temperatur bis auf 95 °C gesteigert und 30 Minuten gehalten. Anschließend wurde wie in Beispiel 12 beschrieben weiterverfahren.

Ausbeute : Produkt feucht 346,0 g (95,2 % der Theorie) ;
Gehalt : 92,5 %iges 3-Nitro-4-aminoanisol.

Nach dem Trocknen des Feuchtproduktes bis zur Gewichtskonstanz im Vakuum bei 60 bis 70 °C weist das Produkt eine Reinheit von 99,7 % auf.

## Beispiel 14

Es wurde wie in Beispiel 12 verfahren, jedoch wurden anstelle von 4,6 g 2,3 g einer 30 %igen (0,01 Mol) wäßrigen Natriumnitritlösung vorgelegt. Nach beendetem Eintropfen und 30 minütigem Nachrühren unter Rückfluß wird die erhaltene Lösung mit 200 ml Wasser versetzt. Bei 30 bis 35 °C werden die Phasen getrennt und anschließend läßt man unter Rühren die untere Methylenchloridphase bei ca. 50 bis 60 °C in verdünnte Schwefelsäure bestehend aus 1 764 ml Wasser und 196 g 100 %ige (2 Mol) Schwefelsäure, welche noch zusätzlich 9,7 g (0,1 Mol) Amidosulfonsäure enthält, einfließen, wobei Methylenchlorid mit etwas Wasser abdestilliert. Anschließend wird die Temperatur bis auf 95 °C gesteigert und 3,5 Stunden gehalten, wobei die Acetylaminogruppe zur Aminogruppe hydrolysiert. Danach wird mit 1 960 ml Wasser versetzt, auf 40 °C abgekühlt, 1 Stunde bei 40 °C nachgerührt, abgesaugt und mit Wasser säurefrei gewaschen. Das Feuchtprodukt wird bis zur Gewichtskonstanz im Vakuum bei 60 bis 70 °C getrocknet.

Ausbeute : Produkt trocken 312,5 g (92,4 % der Theorie).
Reinheit : 99,4 %iges 3-Nitro-4-aminoanisol.

Durch Abstumpfen des pH-Wertes der Mutterlauge mit gasförmigem Ammoniak auf pH 5 und anschließender Filtration bei 20 °C lassen sich weitere 2 % der Theorie an 3-Nitro-4-aminoanisol isolieren.

## Beispiel 15

In einem 4 l Vierhalskolben, ausgerüstet mit Rührer, Rückflußkühler, Thermometer und zwei Tropftrichtern werden 1 400 ml Methylenchlorid und 28,3 g 98 %ige (0,44 Mol) Salpetersäure vorgelegt, unter Normaldruck zum Sieden erhitzt und innerhalb von 3 Stunden 254,6 g 98 %ige (3,96 Mol) Salpetersäure und 329,3 g (2 Mol) 99 %iges technisches 2,4-Dimethylacetanilid, gelöst in 1 200 ml Methylenchlorid simultan zugetropft. Nach beendetem Eintropfen wird noch 30 Minuten unter Rückfluß erhitzt und die erhaltene Lösung mit 200 ml Wasser versetzt. Bei 30 bis 35 °C werden die Phasen getrennt und anschließend läßt man unter Rühren die Methylenchloridphase bei ca. 50 bis 60 °C in verdünnte Natronlauge, bestehend aus 793 ml Wasser und 304 g (7,6 Mol) NaOH einfließen gelassen, wobei Methylenchlorid mit etwas Wasser abdestilliert. Anschließend wird die Temperatur bis auf 80 °C gesteigert, der Rest Methylenchlorid bei vermindertem Druck entfernt, ebenfalls bei 80 °C mit 793 g Methanol versetzt und 3,75 Stunden unter Rückfluß erhitzt, wobei die Acetylaminogruppe zur Aminogruppe hydrolysiert. Danach wird auf 25 °C abgekühlt, 0,5 Stunden bei 25 °C nachgerührt, abgesaugt und mit Wasser alkalifrei gewaschen. Das Feuchtprodukt wird bis zur Gewichtskonstanz im Vakuum bei 50 bis 55 °C getrocknet.

Ausbeute : Produkt trocken 278,3 g (83,3 % der Theorie).
Reinheit : 99,5 %iges 4,6-Dimethyl-2-nitroanilin.

Beispiel 16

In einem 2 l Vierhalskolben, ausgerüstet mit Rührer, Rückflußkühler, Thermometer und zwei Tropftrichtern werden 694 ml Methylenchlorid, 14,8 g 98 %ige (0,23 Mol) Salpetersäure und 2,7 g 30 %ige (0,011 7 Mol) wäßrige Natriumnitritlösung vorgelegt, unter Normaldruck zum Sieden erhitzt und innerhalb von 2 Stunden 134,4 g 98 %ige (2,09 Mol) Salpetersäure und 309,9 g (1,16 Mol) 94,8 %iges N-[[2-[2-(Methoxyethoxy)]-ethoxyphenyl]]-acetamid simultan zugetropft. Nach beendetem Eintropfen wird noch 30 Minuten unter Rückfluß erhitzt, die erhaltene Lösung mit 116 ml Wasser versetzt, unter Rückfluß mit einer 45 %igen wäßrigen Natronlauge auf pH 5 gestellt und auf 30 °C abgekühlt. Bei 30 °C werden die Phasen getrennt und anschließend läßt man unter Rühren die Methylenchloridphase bei ca. 50 bis 60 °C in verdünnte Schwefelsäure, bestehend aus 682,5 ml Wasser und 227,5 g 100 %ige (2,32 Mol) Schwefelsäure, welche noch zusätzlich 4,5 g (0,046 3 Mol) Amidosulfonsäure enthält, einfließen, wobei Methylenchlorid mit etwas Wasser abdestilliert. Anschließend wird die Temperatur bis auf 80 °C gesteigert, der Rest Methylenchlorid bei vermindertem Druck entfernt und die Temperatur von 80 °C 1,25 Stunden gehalten, wobei die Acetylaminogruppe zur Aminogruppe hydrolysiert. Danach wird auf 40 °C gekühlt, bei externer Kühlung mit einer 45 %igen wäßrigen Natronlauge auf pH 4 gestellt und die obere ölige Phase bei 40 °C von der Natriumsulfatlösung abgetrennt. Zur genauen Ausbeutebestimmung wurde die Natriumsulfatlösung einmal mit 100 ml Methylenchlorid extrahiert, die ölige Phase in Methylenchlorid aufgenommen, die vereinigten Methylenchloridlösungen über Natriumsulfat getrocknet, das Methylenchlorid im Vakuum entfernt und das Öl im Vakuum bei 60 °C bis zur Gewichtskonstanz getrocknet.

Ausbeute: Produkt trocken 287,6 g (82,1 % der Theorie). Zur Reinheitsbestimmung wurde ein Teil des Produktes in Essigester aufgenommen und über eine Kieselgelsäule filtriert. Die erhaltene Lösung wurde im Vakuum bis zur Gewichtskonstanz eingedampft und es wurden 97,9 % des eingesetzten Produktes erhalten.

Reinheit : 86,7 %iges 2-[2-(2-Methoxyethoxy)]-ethoxy-4-nitroanilin, welches noch 10,1 % 2,5-Isomeres enthält.

Wird nach erfolgter Spaltung nicht mit NaOH auf pH 4 gestellt, sondern mit Wasser verdünnt und bei 20 °C aus einer 5 gew.-%igen Schwefelsäure, bzw. nach Abstumpfen der Lösung mit gasförmigem Ammoniak bei pH 0,5 isoliert, so weisen die erhaltenen Produkte Reinheiten von 98,1 bzw. 95,4 % auf und enthalten nur 0,6 bzw. 2,2 % 2,5-Isomeres.

Beispiel 17

(Nacharbeitung des Beispiels 2 der US-PS 2 459 002)

Der Versuch wurde in einem 1 Mol Ansatz wie in Beispiel 2 der US-PS beschrieben als Eintopfverfahren ohne Zwischenisolierung des 2-Acetylaminoanisols mit anschließender Nitrierung durchgeführt, wobei das 5-Nitro-2-acetylaminoanisol

a) bei 30 °C
b) bei 20 °C

isoliert, mit Wasser säurefrei und bis zur Gewichtskonstanz im Vakuum bei 60 °C getrocknet wurde.

a) Ausbeute : Produkt trocken 67,3 g (31,0 % der Theorie).
Reinheit : 96,9 %ig.
Schmelzpunkt : 150-153 °C.
b) Ausbeute : Produkt trocken 120,2 g (54,8 % der Theorie).
Reinheit : 95,9 %ig.
Schmelzpunkt : 145-152 °C.

Wegen der geringen Ausbeuten an zwischenisoliertem 5-Nitro-2-acetylaminoanisol wurde die anschließende Entacetylierung nicht durchgeführt.

Beispiel 18

(Nacharbeitung des Beispiels 2 der US-PS 2 459 002)

Der Versuch wurde in einem 2 Mol Ansatz wie im Beispiel 2 der US-PS beschrieben als Eintopfverfahren jedoch ohne Zwischenisolierung des 5-Nitro-2-acetylaminoanisols wie im Beispiel 4 der US-PS beschrieben mit anschließender Neutralisation der überschüssigen Salpetersäure mit Soda, Wasserdampfdestillation des Chlorbenzols, schwefelsaurer Entacetylierung und Klärung mit Aktivkohle durchgeführt. Die schwefelsaure Lösung wurde in 5 l Eiswasser eingerührt, das ausgefallene Produkt abgesaugt, mit Wasser säurefrei gewaschen und bis zur Gewichtskonstanz im Vakuum bei 60 bis 70 °C getrocknet.

Ausbeute : Produkt trocken 91,6 g (15,0 % der Theorie).
Reinheit : 55,1 %iges 5-Nitro-2-aminoanisol.

Die Mutterlauge wurde mit gasförmigem Ammoniak auf pH 7 bis 8 abgestumpft, das ausgefallene Produkt abgesaugt, mit Wasser gewaschen und bis zur Gewichtskonstanz im Vakuum bei 60 bis 70 °C getrocknet.

Ausbeute : Produkt trocken 132,5 g (32,9 % der Theorie).
Reinheit : 83,5 %iges 5-Nitro-2-aminoanisol.

## Ansprüche

1. Verfahren zur Herstellung von Nitroaminobenzolen durch Nitrierung von am Stickstoff geschützten Aminobenzolen mit Salpetersäure in Gegenwart von inerten, mit Wasser nicht mischbaren organischen Lösungs- und/oder Verdünnungsmitteln, gegebenenfalls in Gegenwart von salpetriger Säure und/oder Salpetrigsäure bildenden Substanzen sowie gegebenenfalls in Gegenwart von Wasser bindenden Mitteln, bei Temperaturen von 0 bis 80 °C, dadurch gekennzeichnet, daß man unter gleichzeitiger Zugabe von 50 bis 100 gew.-%iger Salpetersäure und der zu nitrierenden Verbindung in Gegenwart von Methylenchlorid nitriert, anschließend die Schutzgruppe der Nitroaminobenzole, gegebenenfalls nach vorheriger Abtrennung des Methylenchlorids, abspaltet und die Nitroaminobenzole isoliert.

2. Verfahren nach Anspruch 1, dadurch gekennzeichnet, daß man nach beendeter Nitrierung die überschüssige Salpetersäure ganz oder teilweise aus dem Nitriergemisch, gegebenenfalls nach vorheriger Neutralisation der Salpetersäure, entfernt, anschließend die Schutzgruppe der Nitroaminobenzole, gegebenenfalls nach vorheriger Abtrennung des Methylenchlorids, abspaltet und danach die Nitroaminobenzole isoliert.

3. Verfahren nach Ansprüchen 1 und 2, dadurch gekennzeichnet, daß man die überschüssige Salpetersäure ganz oder teilweise aus dem Nitriergemisch, gegebenenfalls nach vorheriger Neutralisation der Salpetersäure, durch Extraktion mit Wasser entfernt.

4. Verfahren nach Ansprüchen 1 bis 3, dadurch gekennzeichnet, daß man die Ausgangsverbindung und die Salpetersäure gleichzeitig einem vorgelegten Gemisch aus Methylenchlorid und Salpetersäure zugibt.

5. Verfahren nach Ansprüchen 1 bis 4, dadurch gekennzeichnet, daß man pro Mol Ausgangsverbindung 1,1 bis 9 Mol einer 100 gew.-%igen Salpetersäure oder 1,3 bis 15 Mol einer 50 gew.-%igen Salpetersäure einsetzt und für Salpetersäurekonzentrationen zwischen 50 und 100 Gew.-% das günstigste Molverhältnis von Salpetersäure zu Ausgangsverbindung ermittelt.

6. Verfahren nach Ansprüchen 1 bis 5, dadurch gekennzeichnet, daß man pro Mol Ausgangsverbindung 400 bis 2 500 ml Methylenchlorid einsetzt.

7. Verfahren nach Ansprüchen 1 bis 6, dadurch gekennzeichnet, daß man die Schutzgruppe der Nitroaminobenzole im sauren Medium abspaltet.

8. Verfahren nach Ansprüchen 1 bis 6, dadurch gekennzeichnet, daß man die Schutzgruppe der Nitroaminobenzole in alkalischem Medium abspaltet.

9. Verfahren nach Ansprüchen 1 bis 8, dadurch gekennzeichnet, daß man als am Stickstoff geschützte Aminobenzole solche der allgemeinen Formel

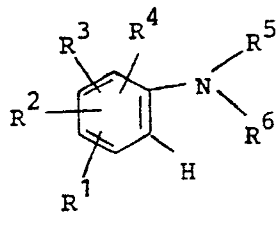

einsetzt, worin

$R^1$ bis $R^4$ gleich oder verschieden sind und für 1 bis 4 Wasserstoffatome, 1 bis 4 Halogenatome, 1 bis 4 Alkylreste, 1 bis 2 Alkoxyreste, 1 bis 2 Acyloxyreste, 1 bis 2 Aryloxyreste und für einen Acylamino-, einen Carboxyl-, einen Alkoxycarbonyl-, einen Alkylsulfonyl-, einen Aralkoxy-, einen Aralkylsulfonyl-, einen Arylsulfonyl-, einen Hydroxysulfonyl-, oder einen Aminosulfonylrest stehen

$R^5$ Wasserstoff und

$R^6$ Formyl, Oxalyl, Alkylcarbonyl, Alkylsulfonyl, Alkoxycarbonyl, Arylcarbonyl, Arylsulfonyl oder

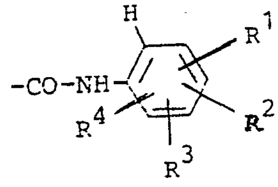

worin

$R^1$ bis $R^4$ die obengenannte Bedeutung haben, oder

—CO—NH—R'

wobei

R' Wasserstoff oder Alkyl sein kann, bedeuten und wobei

$R^5$ und $R^6$ für einen Phthalylrest stehen und wobei

$R^5$ und $R^6$ gemeinsam mit einem zum durch die geschützte Aminogruppe substituierten Kohlenstoffatom ortho-ständigen Ringkohlenstoffatom einen 2-methylsubstituierten Oxazol-, einen Oxazolon- oder einen 2,6-Dioxo-dihydro-1,3-oxazinring bilden, wobei gleichzeitig in para-Stellung zu dem Ringkohlenstoffatom, das durch die geschützte Aminogruppe substituiert ist, einer der Reste $R^1$ bis $R^4$ außer Wasserstoff vorhanden sein muß.

10. Verfahren nach Ansprüchen 1 bis 8, dadurch gekennzeichnet, daß man als am Stickstoff geschützte Aminobenzole solche der allgemeinen Formel

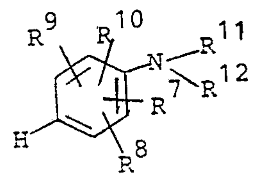

einsetzt, worin

$R^7$ bis $R^{10}$ gleich oder verschieden sind und für 1 bis 4 Wasserstoffatome, 1 bis 4 Halogenatome, 1 bis 4 Alkylreste, 1 bis 2 Alkoxyreste, 1 bis 2 Acyloxyreste, 1 bis 2 Aryloxyreste, einen Acylamino-, einen Carboxyl-, einen Alkoxycarbonyl-, einen Alkylsulfonyl-, einen Aralkoxy-, einen Aralkylsulfonyl-, einen Arylsulfonyl-, einen Hydroxysulfonyl- oder einen Aminosulfonylrest stehen

$R^{11}$ Wasserstoff und

$R^{12}$ Formyl, Oxalyl, Alkylcarbonyl, Alkylsulfonyl, Alkoxycarbonyl, Arylcarbonyl, Arylsulfonyl oder

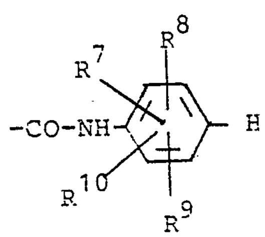

wobei

$R^7$ bis $R^{10}$ die obengenannte Bedeutung haben oder

—CO—NH—R'

wobei

R' für Wasserstoff oder Alkyl steht, bedeuten und wobei

$R^{11}$ und $R^{12}$ für einen Phthalylrest stehen und wobei

$R^{11}$ und $R^{12}$ gemeinsam mit einem durch die geschützte Aminogruppe substituierten Kohlenstoffatom ortho-ständigen Ringkohlenstoffatom einen 2-methylsubstituierten Oxazol-, einen Oxazolon- oder einen 2,6-Dioxo-dihydro-1,3-oxazinring bilden.

## Claims

1. Process for the preparation of nitroaminobenzenes by nitrating aminobenzenes, which are protected at the nitrogen, with nitric acid in the presence of inert, water-immiscible organic solvents and/or diluents, optionally in the presence of nitrous acid and/or of substances which form nitrous acid, and optionally in the presence of water-binding agents, at temperatures of 0 to 80 °C, characterised in that the nitration is carried out in the presence of methylene chloride, with simultaneous addition of 50 to 100 % strength by weight nitric acid and of the compound to be nitrated, the protective group of the nitroaminobenzenes is then split off, optionally after first removing the methylene chloride, and the nitroaminobenzenes are isolated.

2. Process according to Claim 1, characterised in that after completion of nitration the excess nitric

acid is removed, completely or partially from the nitration mixture, optionally after prior neutralisation of the nitric acid, thereafter the protective group of the nitroaminobenzenes is split off, optionally after prior removal of the methylene chloride, and the nitroaminobenzenes are subsequently isolated.

3. Process according to Claims 1 and 2, characterised in that the excess nitric acid is removed, completely or partially from the nitration mixture, optionally after prior neutralisation of the nitric acid, by means of extraction with water.

4. Process according to Claims 1 to 3, characterised in that the starting compound and the nitric acid are added simultaneously to an initial mixture of methylene chloride and nitric acid.

5. Process according to Claims 1 to 4, characterised in that per mol of starting compound 1.1 to 9 mols of a 100 % strength by weight nitric acid or 1.3 to 15 mols of a 50 % strength by weight nitric acid are employed and for nitric acid concentrations which lie between 50 and 100 % by weight the most advantageous molar ratio of nitric acid to starting compound is determined.

6. Process according to Claims 1 to 5, characterised in that 400 to 2,500 ml of methylene chloride are employed per mol of starting compound.

7. Process according to Claims 1 to 6, characterised in that the protective group of the nitroaminobenzenes is split off in an acid medium.

8. Process according to Claims 1 to 6, characterised in that the protective group of the nitroaminobenzenes is split off in an alkaline medium.

9. Process according to Claims 1 to 8, characterised in that the aminobenzenes, protected at the nitrogen, which are employed are those of the general formula

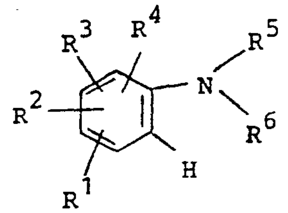

wherein

$R^1$ to $R^4$ are identical or different and represent 1 to 4 hydrogen atoms, 1 to 4 halogen atoms, 1 to 4 alkyl radicals, 1 to 2 alkoxy radicals, 1 to 2 acyloxy radicals, 1 to 2 aryloxy radicals, an acylamino radical, a carboxyl radical, an alkoxycarbonyl radical, an alkylsulphonyl radical, an aralkoxy radical, an aralkylsulphonyl radical, an arylsulphonyl radical, a hydroxysulphonyl radical or an aminosulphonyl radical,

$R^5$ denotes hydrogen and

$R^6$ denotes formyl, oxalyl, alkylcarbonyl, alkylsulphonyl, alkoxycarbonyl, arylcarbonyl, arylsulphonyl or

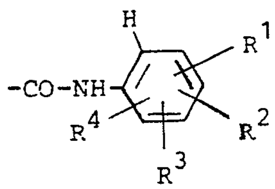

wherein

$R^1$ to $R^4$ have the abovementioned meaning, or

$R^6$ denotes

$$—CO—NH—R'$$

wherein

R' is hydrogen or alkyl, or

$R^5$ and $R^6$ represent a phthalyl radical or

$R^5$ and $R^6$ conjointly with a ring carbon atom which is in the ortho-position to the carbon atom substituted by the protected amino group form a 2-methyl-substituted oxazole ring, an oxazolone ring or a 2.6-dioxo-dihydro-1.3-oxazine ring, in which case, at the same time, one of the radicals $R^1$ to $R^4$ other than hydrogen must be present in the para-position to the ring carbon atom which is substituted by the protected amino group.

10. Process according to Claims 1 to 8, characterised in that the aminobenzenes, protected at the nitrogen, which are employed are those of the general formula

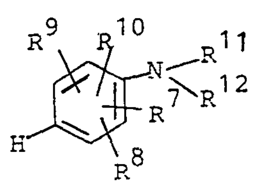

$R^7$ to $R^{10}$ are identical or different and represent 1 to 4 hydrogen atoms, 1 to 4 halogen atoms, 1 to 4 alkyl radicals, 1 to 2 alkoxy radicals, 1 to 2 acyloxy radicals, 1 to 2 aryloxy radicals, an acylamino radical, a carboxyl radical, an alkoxycarbonyl radical, an alkylsulphonyl radical, an aralkoxy radical, an aralkylsulphonyl radical, an arylsulphonyl radical, a hydroxysulphonyl radical or an aminosulphonyl radical,

$R^{11}$ denotes hydrogen and

$R^{12}$ denotes formyl, oxalyl, alkylcarbonyl, alkylsulphonyl, alkoxycarbonyl, arylcarbonyl, arylsulphonyl or

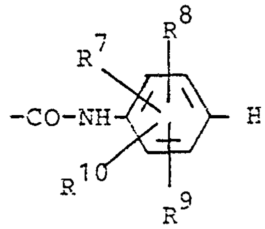

wherein

$R^7$ to $R^{10}$ have the abovementioned meaning, or

$R^{12}$ denotes

$$—CO—NH—R'$$

wherein

R' represents hydrogen or alkyl, or

$R^{11}$ and $R^{12}$ represent a phthalyl radical, or

$R^{11}$ and $R^{12}$, conjointly with a ring carbon atom which is in the ortho-position to the carbon atom substitued by the protected amino group form a 2-methyl-substituted oxazole ring, an oxazolone ring or a 2.6-dioxo-dihydro-1.3-oxazine ring.

**Revendications**

1. Procédé de préparation de nitro-amino-benzènes par nitration d'aminobenzènes protégés sur l'atome d'azote avec de l'acide nitrique en présence de diluants et/ou de solvants organiques inertes non miscibles à l'eau, éventuellement en présence d'acide nitreux ou de substances formant de l'acide nitreux, ainsi qu'éventuellement en présence d'agents fixateurs d'eau, à des températures de 0 à 80 °C, caractérisé en ce que, avec addition simultanée d'acide nitrique à 50-100 % en poids et du composé à nitrer, on effectue la nitration en présence de chlorure de méthylène puis, éventuellement après séparation préalable du chlorure de méthylène, on sépare le groupe protecteur des nitroaminobenzènes et on isole les nitro-aminobenzènes.

2. Procédé suivant la revendication 1, caractérisé en ce que, au terme de la nitration, on élimine entièrement ou partiellement l'acide nitrique en excès du mélange de nitration, éventuellement après neutralisation préalable de l'acide nitrique, puis on sépare le groupe protecteur des nitro-amino-benzènes, éventuellement après séparation préalable du chlorure de méthylène et ensuite, on isole les nitro-aminobenzènes.

3. Procédé suivant les revendications 1 et 2, caractérisé en ce que, par extraction avec de l'eau, on élimine entièrement ou partiellement l'acide nitrique en excès du mélange de nitration, éventuellement après neutralisation préalable de l'acide nitrique.

4. Procédé suivant les revendications 1 à 3, caractérisé en ce qu'on ajoute simultanément le composé de départ et l'acide nitrique à un mélange préalablement déposé constitué de chlorure de méthylène et d'acide nitrique.

5. Procédé suivant les revendications 1 à 4, caractérisé en ce que, par mole du composé de départ, on utilise 1,1 à 9 moles d'un acide nitrique à 100 % en poids ou 1,3 à 15 moles d'un acide nitrique à 50 % en poids et, pour des concentrations en acide nitrique se situant entre 50 et 100 % en poids, on détermine le rapport molaire le plus favorable entre l'acide nitrique et le composé de départ.

6. Procédé suivant les revendications 1 à 5, caractérisé en ce que, par mole du composé de départ, on utilise 400 à 2 500 ml de chlorure de méthylène.

7. Procédé suivant les revendications 1 à 6, caractérisé en ce qu'on sépare le groupe protecteur des nitro-aminobenzènes en milieu acide.

8. Procédé suivant les revendications 1 à 6, caractérisé en ce qu'on sépare le groupe protecteur des nitro-aminobenzènes en milieu alcalin.

9. Procédé suivant les revendications 1 à 8, caractérisé en ce que, comme aminobenzènes protégés sur l'atome d'azote, on utilise ceux de formule générale

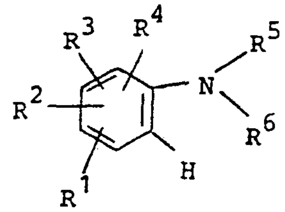

dans laquelle

$R^1$ à $R^4$ sont identiques ou différents et représentent 1 à 4 atomes d'hydrogène, 1 à 4 atomes d'halogènes, 1 à 4 groupes alkyle, 1 ou 2 groupes alcoxy, 1 ou 2 groupes acyloxy, 1 ou 2 groupes aryloxy et un groupe acylamino, un groupe carboxy, un groupe alcoxycarbonyle, un groupe alkylsulfonyle, un groupe aralcoxy, un groupe aralkylsulfonyle, un groupe arylsulfonyle, un groupe hydroxysulfonyle ou un groupe aminosulfonyle,

$R^5$ représente un atome d'hydrogène, et

$R^6$ représente un groupe formyle, un groupe oxalyle, un groupe alkylcarbonyle, un groupe alkylsulfonyle, un groupe alcoxycarbonyle, un groupe arylcarbonyle, un groupe arylsulfonyle ou un groupe

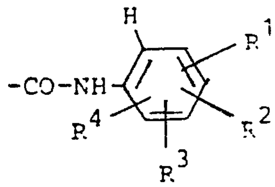

où

$R^1$ à $R^4$ ont les significations indiquées ci-dessus, ou

—CO—NH—R′

où

R′ peut être un atome d'hydrogène ou un groupe alkyle, tandis que

$R^5$ et $R^6$ peuvent représenter un groupe phtalyle et

$R^5$ et $R^6$, ensemble avec un atome de carbone cyclique en position ortho vis-à-vis de l'atome de carbone substitué par le groupe amino protégé, forment un noyau oxazole substitué par un groupe 2-méthyle, un noyau oxazolone ou un noyau 2,6-dioxo-dihydro-1,3-oxazine, un des radicaux $R^1$ à $R^4$, hormis l'hydrogène, devant être simultanément présent en position para par rapport à l'atome de carbone cyclique qui est substitué par le groupe amino protégé.

10. Procédé suivant les revendications 1 à 8, caractérisé en ce que, comme aminobenzènes protégés sur l'atome d'azote, on utilise ceux de formule générale

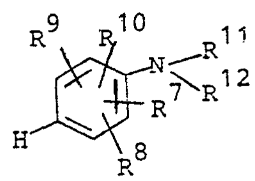

dans laquelle

$R^7$ à $R^{10}$ sont identiques ou différents et représentent 1 à 4 atomes d'hydrogène, 1 à 4 atomes d'halogènes, 1 à 4 groupes alkyle, 1 ou 2 groupes alcoxy, 1 ou 2 groupes acyloxy, 1 ou 2 groupes aryloxy, un groupe acylamino, un groupe carboxy, un groupe alcoxycarbonyle, un groupe alkylsulfonyle, un groupe aralcoxy un groupe aralkylsulfonyle, un groupe arylsulfonyle, un groupe hydroxysulfonyle ou un groupe aminosulfonyle,

$R^{11}$ représente un atome d'hydrogène, et

$R^{12}$ représente un groupe formyle, un groupe oxalyle, un groupe alkylcarbonyle, un groupe alkylsulfonyle, un groupe alcoxycarbonyle, un groupe arylcarbonyle, un groupe arylsulfonyle ou un groupe

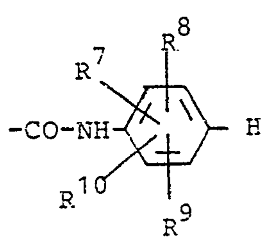

où

$R^7$ à $R^{10}$ ont les significations indiquées ci-dessus, ou

—CO—NH—R'

où

R' représente un atome d'hydrogène ou un groupe alkyle, tandis que,

$R^{11}$ et $R^{12}$ représentent un groupe phtalyle, et

$R^{11}$ et $R^{12}$, ensemble avec un atome de carbone cyclique en position ortho vis-à-vis de l'atome de carbone substitué par le groupe amino protégé, forment un noyau oxazole substitué par un groupe 2-méthyle, un noyau oxazolone ou un noyau 2,6-dioxo-dihydro-1,3-oxazine.